(19) Europäisches Patentamt
European Patent Office
Office européen des brevets

(11) Numéro de publication : **0 014 167**
**B1**

# (12) FASCICULE DE BREVET EUROPÉEN

(45) Date de publication du fascicule du brevet : **22.09.82**

(21) Numéro de dépôt : **80420008.7**

(22) Date de dépôt : **22.01.80**

(51) Int. Cl.³ : **C 07 C149/23**, C 07 C153/01, C 07 D213/83, C 07 D307/68, C 07 D333/24, C 07 D333/38, A 01 N 37/46, A 01 N 43/06, A 01 N 43/40

(54) **Produits dérivés de l'aniline ayant une activité antifongique ainsi que leur procédé de préparation.**

(30) Priorité : **24.01.79 FR 7902580**

(43) Date de publication de la demande : **06.08.80 (Bulletin 80/16)**

(45) Mention de la délivrance du brevet : **22.09.82 Bulletin 82/38**

(84) Etats contractants désignés : **BE CH DE FR GB IT LU NL**

(56) Documents cités : **FR A 2 267 042**

(73) Titulaire : **RHONE-POULENC AGROCHIMIE**
**14-20, rue Pierre Baizet**
**F-69009 Lyon (FR)**

(72) Inventeur : **Abblard, Jean**
**42, Rue Pasteur**
**F-69370 St. Didier au Mont d'Or (FR)**
Inventeur : **Lacroix, Guy**
**332 C Balmont - La Duchère**
**F-69009 - Lyon (FR)**

(74) Mandataire : **Brachotte, Charles et al**
**RHONE-POULENC AGROCHIMIE BP 9163-09**
**F-69263 Lyon Cedex 1 (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée foimée qu'après paiement de la taxe d'opposition (Art. 99(1) Convention sur le brevet européen).

Imprimerie Jouve, 18, rue St-Denis, 75001 Paris, France

EP 0 014 167 B1

Produits dérivés de l'aniline ayant une activité antifongique ainsi que leur procédé de préparation

La présente invention concerne de nouveaux produits soufrés à groupement amide dérivant de l'aniline. L'invention concerne aussi la préparation desdits produits et leur application pour la protection des végétaux, spécialement contre les champignons et les maladies fongiques.

Il est déjà connu (brevet FR-A 7510722) des composés fongicides dérivés de N-acyl N-aryl amino acides. Toutefois, il est souhaitable de pouvoir disposer d'un large éventail de produits fongicides de formules différentes. En effet, les qualités de différents pesticides sont rarement identiques les unes aux autres en sorte qu'il vaut mieux disposer d'un grand nombre de produits si l'on veut faire face à tous les cas d'attaques contre les végétaux.

Les produits composés selon l'invention sont caractérisés en ce qu'ils ont pour formule

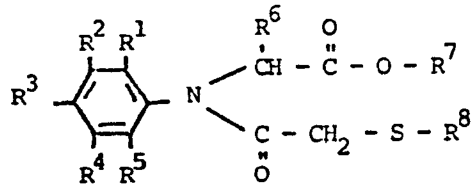

(I)

dans laquelle

$R^1$ et $R^5$, identiques ou différents, représentent un radical alkyle ou alkoxyle ayant de 1 à 4 atomes de carbone ou un atome d'halogène ou l'atome d'hydrogène, un seul des deux radicaux $R^1$ ou $R^5$ pouvant avoir cette dernière signification,

$R^2$, $R^3$ et $R^4$, identiques ou différents, représentent l'atome d'hydrogène ou un atome d'halogène ou un radical alkyle ou alkoxyle ayant de 1 à 4 atomes de carbone,

$R^6$ représente l'atome d'hydrogène ou un radical méthyle,

$R^7$ représente l'atome d'hydrogène ou un radical alkyle ayant de 1 à 12 atomes de carbone ou 1/n $M^{n+}$, M étant un cation de valence n, plus particulièrement un cation d'ammonium (éventuellement substitué) ou un cation métallique,

$R^8$ représente l'atome d'hydrogène ou un radical de formule $R^9$—CO— dans laquelle $R^9$ est un radical organique qui représente

— un radical alkyle ou alkényle, linéaire ou ramifié, ayant au plus 18 atomes de carbone et pouvant être substitué par des atomes d'halogènes ou des groupements hydroxy, mercaptan, cyano, oxo ou des radicaux alkyloxy, alkylthio, aryle, alkoxycarbonyle, acylamino, ces divers radicaux ayant au plus 6 atomes de carbone.

— un radical cycloalkyle comportant 3 à 7 atomes de carbone dans le cycle (de préférence les radicaux cyclopropyle, cyclopentyle, ou cyclohexyle) et pouvant être substitué par des atomes d'halogène ou des groupements alkyle ayant au olus 4 atomes de carbone.

— un radical phényle ou naphtyle éventuellement substitué par des atomes d'halogène ou des groupements alkyle, ou acyloxyle, acyle, alkoxyle, ces divers groupements ayant au plus 6 atomes de carbone.

— un radical hétérocyclique comportant, dans le cycle, 5 ou 6 atomes dont 1 à 3 sont des hétéroatomes d'oxygène, d'azote ou de soufre, ce radical hétérocyclique pouvant être substitué par des atomes de chlore ou des groupements méthyle ou éthyle, et la valence libre de ce radical $R^9$ (c'est-à-dire la valence rattachée au groupe carbonyle dans le radical $R^9$—CO—) étant porté par un atome de carbone.

— un radical comprenant un groupe hétérocyclique ayant la même signification que celle indiquée dans le paragraphe précédent, ce groupe hétérocyclique étant relié au groupe carbonyle (de $R^9$—CO—) par l'intermédiaire d'un radical divalent méthylène ou éthylène.

La substitution d'un radical par un groupe oxo (c'est-à-dire par = O), correspond, selon la terminologie habituelle, à la présence d'un groupe carbonyle —CO— dans ce radical.

Parmi les atomes d'halogène pouvant figurer dans les substances représentées par la formule (I), on peut utiliser notamment le chlore et le brome.

Selon la définition donnée plus haut, le groupe $-\underset{\underset{O}{\|}}{C}-O-R^7$ représente donc un groupe carboxylique ou carboxylate, la liaison O—$R^7$ pouvant être soit de type covalent (notamment dans le cas où —COO—$R^7$ représente un groupe ester) soit de type ionique (notamment dans le cas où —CCO—$R^7$ représente un groupement salin) soit des deux types (notamment dans le cas de —COOH qui peut être ionisé ou non ionisé, ou partiellement ionisé).

Lorsque le groupe —COO—$R^7$ représente un groupement salin, il a été indiqué que $R^7$ peut alors avoir la signification 1/n $M^{n+}$ M étant un cation de valence n ; le groupe —COO—$R^7$ peut, dans ce cas être représenté aussi sous la forme (plus exacte formellement) —$COO^{\ominus}$, 1/n $M^{n+}$ ; mais ceci est aussi une

formule moyenne ; la représentation encore plus correcte (mais en réalité équivalente) de la molécule de formule (I) est alors

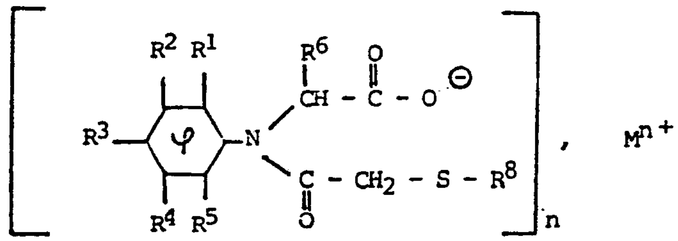

(II)

dans laquelle $R^1$, $R^2$, $R^3$, $R^4$, $R^5$, $R^6$, $R^8$, M et n ont les significations données précédemment.

Dans la famille générale de composés de formule (I) telle que l'on vient de la définir, une sous-famille particulière est spécialement avantageuse ; elle présente notamment de très bonnes propriétés antifongiques spécialement à l'égard des phycomycètes, et plus particulièrement à l'égard des mildious.

Ces produits préférés sont tels que :

$R^2$ représente un atome d'hydrogène, de chlore ou de brome

$R^3$ et $R^4$ représentent l'atome d'hydrogène

$R^1$ et $R^5$ représentent un groupe alcoyle ayant de 1 à 4 atomes de carbone

$R^6$ est l'atome d'hydrogène ou le groupe méthyle

$R^7$ est un groupe alkyle de 1 à 4 atomes de carbone, et plus spécialement le radical méthyle.

Dans ces familles ainsi définies on préfère aussi les composés dans la formule desquels

$R^8$ représente l'atome d'hydrogène ou un radical $R^9$—CO—

$R^9$ représente

— un radical alkyle ou alkényle éventuellement substitués par des atomes de chlore ou des groupes alkoxyle ayant de 1 à 4 atomes de carbone ou des groupes cyano, oxo, acétamido, phényle, chlorophényle, alkylthio, alkoxycarbonyle

— un radical cyclopropyle, cyclopentyle ou cyclohexyle

— un radical phényle éventuellement substitué par un atome de chlore ou de brome

— un radical hétérocyclique furyle, thiényle, trichlorothiényle ou pyridyle.

Les produits dans lesquels $R^1$, $R^5$ et $R^7$ sont le radical méthyle et $R^9$ est un radical alkyle ayant de 1 à 9 atomes de carbone sont tout spécialement avantageux.

Les composés selon l'invention (de formule I) peuvent être préparés selon plusieurs procédés.

Quelques-uns des procédés utilisables sont désignés ci-après respectivement par les lettres (A), (B), (C), (D) et correspondent aux schémas réactionnels suivants :

(Voir Schémas pages 4 et 5)

Procédé (A)

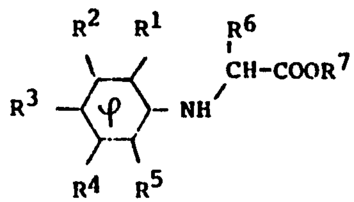

(IV)

+

$Cl-CO-CH_2-S-CO-R^9$

(V)

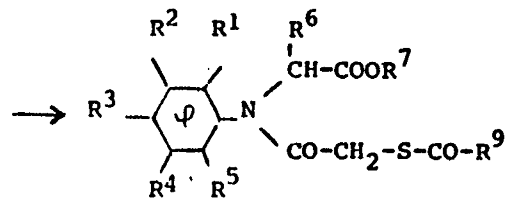

(III)

+ HCl

Procédé (B)

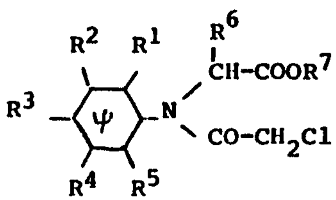

(VI)

+

$M'S-CO-R^9$

(VII bis)

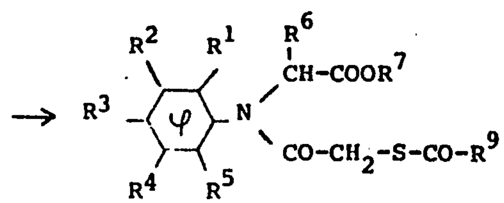

(III)

+ M'Cl

Procédé (C)

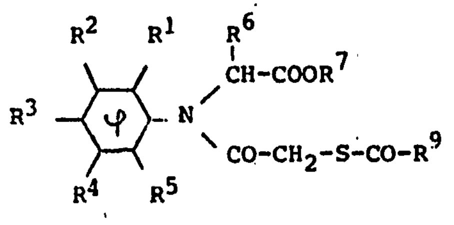

(III)

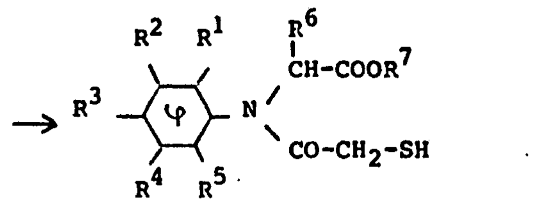

(VIII)

+

alcool ($R^7OH$ par exemple)

+ ester ($R^9COOR^7$ par exemple)

Procédé (D)

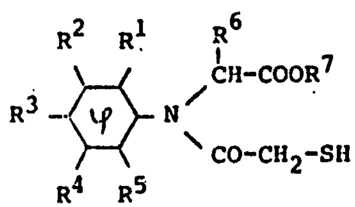

(VIII)

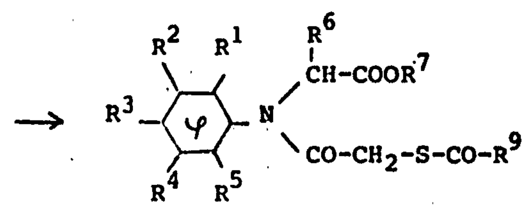

(III)

+

$R^9$ - CO-Cl

ou $(R^9CO)_2O$

Selon le premier procédé, ou procédé (A), on obtient un composé de formule (III) par réaction d'un dérivé de N-phénylaminoacide de formule (IV) avec un chlorure d'acide de formule (V), les symboles $R^1$ à $R^6$ et $R^8$ et $R^9$ ayant les significations données ci-avant et le symbole $R^7$ représentant l'atome d'hydrogène ou un radical alkyle.

La réaction mise en œuvre dans ce procédé (A) s'effectue avantageusement en solution dans un solvant inerte (c'est-à-dire ne réagissant pas chimiquement avec les réactifs dans les conditions opératoires). La température de réaction est généralement supérieure à 40 °C et inférieure à la température de dégradation des réactifs et/ou produits de réaction. La température est ainsi généralement comprise entre 60 et 150 °C. Selon une modalité commode on opère à la température d'ébullition du solvant considéré. Comme solvant approprié on peut citer les solvants organiques aprotiques polaires ou non polaires. On préfère utiliser les solvants dont le point d'ébullition est compris dans la zone indiquée pour la température de réaction ; il peut donc s'agir d'hydrocarbures aromatiques (tels que le benzène, le toluène, les xylènes, le chlorobenzène) ou aliphatiques (tels que l'hexane, l'heptane, le cyclohexane, le méthylcyclohexane), ou d'hydrocarbures aliphatiques chlorés (tels que le dichloroéthane, le dichloroéthylène, le chloroforme, le tétrachlorure de carbone) ou d'éthers (tels que le dioxanne, le tétrahydrofuranne, l'éther diéthylique) ou de cétones (telles que l'acétone, la méthyléthylcétone, la méthylisobutylcétone) ou de nitriles (tel que l'acétonitrile).

La réaction peut être catalysée, par exemple par du diméthylformamide ; elle peut être effectuée en absence ou en présence d'agent de condensation, notamment d'accepteur d'acide. Comme accepteurs d'acide on peut utiliser des amines tertiaires telles que les trialkylamines (par exemple la triéthylamine) ou les N-aryldialkylamines (par exemple la N,N-diméthylaniline) ou encore la pyridine et les bases pyridiniques ou les bases minérales telles que les carbonates et hydrogénocarbonates de métaux alcalin ou alcalinoterreux, et l'acétate de sodium. On peut se servir, en outre, comme accepteur d'acide, d'un excès du composé aminé de formule (IV) et c'est là une modalité opérationnelle préférée.

Il est possible d'effectuer la réaction en présence d'un excès de l'un ou l'autre des réactifs. Comme il vient d'être dit, une modalité préférée consiste cependant à opérer en présence d'un excès de composé de formule (IV), au moins pendant la première partie de la réaction. On peut opérer en présence d'un excès de composé de formule (IV) par exemple en introduisant progressivement le chlorure d'acide de formule (V) dans le milieu réactionnel contenant tout ou partie du composé de formule (IV) devant être mis en œuvre, puis l'on poursuit la réaction jusqu'à cessation du dégagement d'acide chlorhydrique ; les quantités de réactifs mis en œuvre globalement au cours de la réaction sont de préférence voisines de la stœchiométrie ; la stœchiométrie est généralement la plus avantageuse ; toutefois le chlorure d'acide de formule (V) peut être mis en œuvre en quantité dépassant la stœchiométrie, par exemple jusqu'à 10 % (en nombre). En fin de réaction on isole le produit de réaction de formule (III) par tout moyen connu en soi, par exemple par distillation du solvant (c'est-à-dire évaporation) et/ou cristallisation du produit dans le milieu.

En ce qui concerne la préparation des composés de formule (IV), elle peut s'effectuer selon des procédés semblables à ceux décrits pour la préparation d'esters anilino alcanecarboxyliques dans les publications suivantes :

J. Org. Chem. 30 p. 4 101-4 104 (1965)

Tetrahedron 1967 p. 487-498

La préparation des chlorures d'acides de formule (V) peut être faite conformément ou semblablement à bien des procédés connus mais spécialement ceux décrits dans

— le brevet des États-Unis d'Amérique n° 2 412 700

— Berichte 46 p. 2 104-2 107 (1913)

— Annalen 602 p. 1-14 (1957).

Selon le second procédé, ou procédé (B), on prépare des composés de formule (III) dans laquelle $R^1$ à $R^7$ et $R^9$ ont les significations données pour la formule (I) ; cette préparation s'effectue par réaction d'un dérivé de N-phénylaminoacide de formule (VI) avec un thiocarboxylate alcalin ou alcalino-terreux (de préférence alcalin) dérivant d'un acide thiocarboxylique de formule $R^9$—CO—S—H (VII). Le cation ammonium est inclus dans les cations alcalins utilisables. On peut représenter ces thiocarboxylates par la formule $R^9$—CO—S—M' [formule (VII bis)], M' représentant le cation alcalin (ou d'ammonium) ou un demi-équivalent de cation alcalinoterreux.

Les dérivés de formule (VI) peuvent être préparés selon des procédés identiques ou similaires à ceux décrits dans le brevet anglais 1 445 387.

Un moyen commode de préparation du thiocarboxylate alcalin consiste évidemment à faire réagir un acide thiocarboxylique avec un dérivé alcalin ou alcalino-terreux, tel qu'un hydroxyde ou carbonate alcalin ou alcalino-terreux. Si la réaction est faite au sein d'un solvant on peut obtenir ainsi directement une solution (ou éventuellement une suspension) du thiocarboxylate de formule (VII bis).

La préparation du composé de formule (III) à partir des composés de formule (VI) et du thiocarboxylate s'effectue généralement en présence d'un solvant inerte (c'est-à-dire inerte chimiquement vis-à-vis des réactifs ou produits de réaction dans les conditions opératoires). La température de réaction et les solvants utilisables sont semblables à ce qui a été défini à propos du procédé (A). Comme solvants, on peut en outre utiliser des amides (tels que le diméthylformamide) et des sulfoxydes (tels que le diméthylsulfoxyde).

En fin de réaction, on sépare (par exemple par filtration) le chlorure alcalin ou alcalino-terreux qui a précipité et on évapore le solvant ; le cas échéant on peut procéder à des purifications habituelles, comme par exemple un lavage à l'eau d'une solution organique du composé de formule (III).

Selon le troisième procédé, ou procédé (C), on prépare des composés à groupe mercaptan libre de formule (VIII) par réaction entre un composé de formule (III) avec un alcool. Lorsque $R^7$ est un groupe alkyle, on utilise avantageusement un alcool de formule $R^7$—OH ; parmi les alcools utilisables, le méthanol est préféré. Au cours de la réaction mise en œuvre dans ce procédé (C), il se forme un ester (par exemple de formule $R^9COOR^7$) en sorte que la réaction est une transestérification au sens large. La réaction s'effectue de préférence en solution alcoolique (méthanolique le cas échéant) en présence d'un agent (ou catalyseur) alcalin (1$^{re}$ variante) ou acide (2$^e$ variante). La température de réaction est généralement comprise entre 0 et 120 °C.

Comme agent (ou catalyseur) alcalin [1$^{re}$ variante du procédé (C)], on préfère utiliser un alcoolate alcalin, tel que le méthylate (aussi appelé méthanolate) de sodium ou de potassium. La quantité d'alcoolate ou méthylate alcalin mise en œuvre est avantageusement voisine de la stœchiométrie (au moins 90 % de la stœchiométrie) par rapport au composé de formule (III) mis en œuvre. On préfère opérer juste à la stœchiométrie ; selon une modalité préférentielle, on ajoute progressivement une solution alcoolique du composé de formule (III) à une solution alcoolique d'alcoolate alcalin. La température est avantageusement comprise entre 10 et 50 °C. En fin de réaction, on acidifie ou, tout au moins, neutralise à l'aide d'un acide pour éliminer le métal alcalin sous forme de sel insoluble. Ce dernier est ensuite séparé, par exemple par filtration ; le composé de formule (VIII) est obtenu par évaporation du méthanol. On peut bien entendu procéder à une purification par les méthodes habituelles, par exemple par lavage à l'eau d'une solution organique du composé de formule (VIII) suivi éventuellement d'une recristallisation.

La réaction entre un alcool, le méthanol par exemple, et le composé de formule (III) peut, comme cela a déjà été dit, s'effectuer en présence d'un agent ou catalyseur acide [2$^e$ variante du procédé (C)], de préférence un acide minéral ou organique fort, par exemple les acides chlorhydrique, sulfurique, paratoluène sulfonique. La quantité de catalyseur acide mise en œuvre est avantageusement comprise entre 0,01 et 1 équivalent acide (ce qui correspond à 0,01 à 1 ion-g $H^+$ ou mole de mono-acide) par mole de composé (III). La réaction est effectuée généralement au-dessus de 50 °C et jusqu'à la température d'ébullition du milieu réactionnel ; on opère pratiquement par simple chauffage en présence du catalyseur acide.

En fin de réaction, on élimine l'alcool, (le méthanol le cas échéant) et le catalyseur acide, par exemple par distillation et, éventuellement, on purifie par les méthodes habituelles.

Selon le quatrième procédé, ou procédé (D), on prépare des composés de formule (III) par réaction d'un mercaptan de formule (VIII) avec un dérivé d'acide $R^9COOH$, de préférence un chlorure d'acide $R^9$—CO—Cl (1$^{re}$ variante) ou un anhydride $(R^9—CO)^2O$ (2$^e$ variante).

La réaction d'un chlorure d'acide $R^9$—CO—Cl avec un mercaptan de formule (VIII), selon la 1$^{re}$ variante, s'effectue généralement en présence d'un agent de condensation qui le plus souvent est un accepteur d'acide (également appelé accepteur basique) ; on opère habituellement au sein d'un solvant organique inerte.

Comme solvant organique inerte on peut citer ceux indiqués comme souhaitables dans le cas du procédé (A).

Comme accepteur d'acide, on peut utiliser les bases organiques comme les amines tertiaires telles que les trialkylamines (par exemple la triéthylamine) ou les N-aryldialkylamines (par exemple la N,N-diméthylaniline), ou encore la pyridine et les bases pyridiniques, ou les bases minérales telles que les carbonates ou hydrogénocarbonates alcalins ou alcalino-terreux, ou l'acétate de sodium.

La réaction s'effectue le plus commodément à température ambiante, mais des températures de 5 à 60 °C, de préférence de 10 à 30 °C peuvent aussi être utilisées.

Les proportions des différents réactifs sont avantageusement voisines (au moins 90 %) de la stœchiométrie ; la stœchiométrie exacte (100 %) est préférée.

La réaction s'effectue préférentiellement par mélange du chlorure d'acide (éventuellement à l'état de solution) avec un mélange du composé de formule (VIII) et de l'accepteur d'acide (ce dernier mélange étant éventuellement à l'état de solution). Selon la modalité la plus avantageuse, on ajoute progressivement le chlorure d'acide au mélange des deux autres réactifs.

Au cours de la réaction, l'accepteur d'acide donne naissance habituellement, avec l'acide qui se forme pendant la réaction, à un sous-produit de réaction qui peut être un composé d'addition, par exemple un chlorhydrate. Il peut s'agir d'un sous-produit de réaction insoluble ou soluble dans le milieu. En fin de réaction ce sous-produit de réaction est séparé ; si ce sous-produit de réaction est insoluble, la séparation peut être effectuée par exemple par filtration ou essorage ; si ce sous-produit de réaction est soluble dans le milieu, la séparation peut être effectuée par exemple par lavage à l'eau du milieu réactionnel. D'une manière ou de l'autre, on procède ensuite à une élimination (généralement par évaporation) du solvant qui conduit au composé de formule (III) ; on peut bien entendu procéder également aux purifications habituelles, par exemple par lavage d'une solution organique de (III) avec de l'eau ou avec des solutions aqueuses acides, et/ou par recristallisation.

Selon la 2$^e$ variante du procédé (D) on fait réagir un anhydride d'acide $(R^9—CO)^2O$ avec un mercaptan de formule (VIII). Selon une modalité avantageuse l'anhydride est préparé in situ par action

d'un agent de déshydratation sur l'acide $R^9COOH$.

La réaction s'effectue le plus commodément à des températures comprises entre 10 et 120 °C, dans un solvant organique inerte. Comme solvant utilisable on peut citer les solvants utilisables dans le procédé (A).

Comme agent de déshydratation on peut citer les carbodiimides, notamment le dicyclohexylcarbodiimide. Les quantités d'agent de déshydratation et d'acide $R^9COOH$ sont de préférence voisines de la stœchiométrie (au moins 90 % de la stœchiométrie) par rappoort au mercaptan de formule (VIII).

Sur un plan pratique on préfère aussi ajouter progressivement le carbodiimide à un mélange de mercaptan de formule (VIII) et d'acide $R^9COOH$.

Au cours de la réaction il se forme généralement une urée substituée qui s'élimine en fin de réaction par tout moyen connu en soi, par exemple par filtration lorsque cette urée substituée est insoluble. Le solvant est également éliminé, par exemple par évaporation, ce qui conduit à l'isolement du composé de formule (III).

D'autres procédés de préparations particulièrement adaptés aux produits selon l'invention sous forme sel peuvent encore être utilisés.

Ces composés de formule (II) peuvent être préparés par exemple par saponification des esters correspondants (produits de formule (III) dans lesquels $R^7$ est un radical alkyle et $R^8$ est l'atome d'hydrogène), cette saponification étant faite par exemple à l'aide d'hydroxydes alcalins ou alcalino-terreux. Les composés de formule (II) peuvent aussi être obtenus (spécialement lorsque $R^8$— représente un radical $R^9$—CO—) par réaction d'un hydroxyde alcalin ou alcalino-terreux avec un composé de formule (II) dans laquelle $R^7$ est l'atome d'hydrogène. On peut encore utiliser des réactions de double décomposition entre un sel soluble de formule (II) et un sel minéral soluble du cation recherché.

Les exemples suivants, donnés à titre non limitatif, illustrent l'invention et montrent comment elle peut être mise en pratique.

Les exemples 1 à 10 illustrent la préparation de composés selon l'invention selon les divers procédés utilisables décrits ci-avant.

Les tableaux I, II et III donnent les caractéristiques physiques et l'analyse élémentaire de différents produits selon l'invention préparés selon des procédés analogues à l'un ou l'autre des procédés figurant dans les exemples 1 à 10. Lorsque les produits obtenus étaient cristallisés, on a indiqué leur point de fusion ; lorsque les produits étaient huileux, on a indiqué l'indice de réfraction ($n_D^{20}$) mesuré à 20 °C pour la raie D du sodium.

Les propriétés pesticides des produits des tableaux I, II et III sont illustrées dans les exemples 11 à 14 ; dans les conditions de ces exemples, aucun composé testé n'a présenté de phytotoxicité.

Dans les exemples 11 à 14 on considère qu'un produit exerce une protection totale vis-à-vis d'une maladie fongique lorsque la protection est d'au moins 95 % ; la protection est considérée comme bonne lorsqu'elle est d'au moins 80 % (mais inférieure à 95 %), comme assez bonne lorsqu'elle est d'au moins 70 % (mais inférieure à 80 %), comme moyenne lorsqu'elle est d'au moins 50 % (mais inférieure à 70 %).

Dans le présent exposé les pourcentages sont, sauf indication contraire et sauf ceux concernant les rendements, des pourcentages pondéraux. Dans le cas où les pourcentages sont exprimés par rapport à la stœchiométrie, il s'agit de pourcentages molaires.

Les composés 73 et 80 ont été représentés sous forme ionique ; il doit être bien entendu qu'ils peuvent aussi être considérés comme étant sous la forme covalente —COOH.

Dans les exemples qui suivent la structure de tous les produits obtenus a été vérifiée par spectrographie de résonance magnétique nucléaire (R.M.N.).

## Exemple 1

Dans 40 $cm^3$ de toluène, on dissout 10,35 g d'ester méthylique de la N-(2,6-diméthylphényl) alanine, de formule

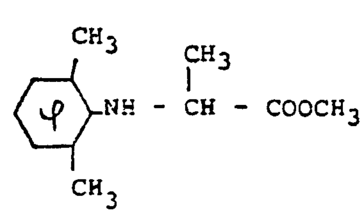

Cette solution est chauffée jusqu'à l'ébullition. Dans cette solution ainsi chauffée à l'ébullition à reflux, on ajoute progressivement en 10 mn une seconde solution constituée de 20 $cm^3$ de toluène et 7,63 g de chlorure d'acétylthioacétyle de formule $CH_3$—CO—S—$CH_2$—CO—Cl. On observe un dégagement d'acide chlorhydrique durant cette opération, et le chauffage est poursuivi jusqu'à cessation de ce dégagement gazeux (c'est-à-dire durant 15 mm). On élimine alors le toluène par distillation sous pression réduite.

On obtient ainsi, avec un rendement de 100 %, l'ester méthylique de la N-[(acétylthio) acétyl]-N-(2,6-diméthylphényl) alanine qui est le composé n° 1 du tableau (I).

Son point de fusion après recristallisation dans l'alcool éthylique ainsi que l'analyse élémentaire sont indiqués dans le tableau I.

Exemple 2

Une solution de 19,3 g (0,1 mole) de N-(2,6-diméthylphényl) alanine dans 100 $cm^3$ de toluène est chauffée à ébullition à reflux. Tout en maintenant l'ébullition, on ajoute progressivement en 15 mn une solution constituée de 15,25 g (0,1 mole) de chlorure d'acétylthioacétyle et de 20 $cm^3$ de toluène. L'ébullition est ensuite maintenue encore 15 mn jusqu'à cessation du dégagement d'acide chlorhydrique gazeux. La moitié (en volume) des solvants du milieu réactionnel est éliminée par distillation ; on refroidit à 0 °C ; le précipité obtenu est filtré, lavé avec 10 $cm^3$ d'hexane, essoré et enfin séché sous vide (pression absolue réduite à 27 millibars) à 20 °C. On obtient ainsi 15,5 g (rendement : 55 %) de N-[(acétylthio) acétyl]-N-(2,6-diméthylphényl) alanine (composé n° 80) dont le point de fusion et l'analyse élémentaire sont indiqués au tableau III.

La réaction effectuée a été :

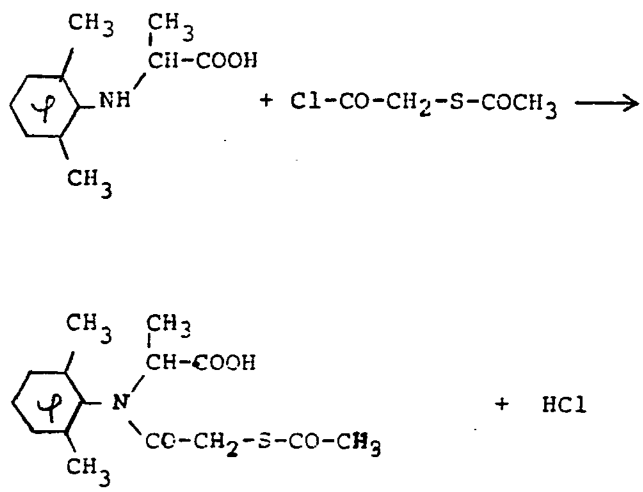

Exemple 3

4,18 g d'acide mercaptoacétique ($CH_3$ CO SH) sont coulés en 30 mn sur une suspension de 2,97 g de carbonate de sodium dans 30 $cm^3$ de diméthylformamide. Le milieu étant devenu homogène, on ajoute 14,17 g d'ester méthylique de la N-(2,6-diméthyl phényl)-N-(chloroacétyl) alanine de formule :

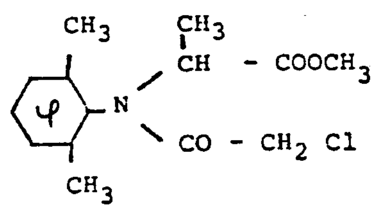

Le mélange est chauffé 2 heures à 100 °C sous agitation. Après refroidissement, le chlorure de sodium formé est éliminé par filtration et le diméthylformamide est éliminé par distillation sous pression réduite. L'huile résiduelle est dissoute dans 30 $cm^3$ d'eau chaque fois ; la solution organique ainsi lavée est séchée sur sulfate de magnésium, filtrée, puis concentrée jusqu'à siccité. L'huile obtenue, additionnée de 5 $cm^3$ d'éthanol, fournit un précipité cristallisé qui se forme lentement. On obtient ainsi, avec un rendement de 62 %, l'ester méthylique de la N-(2,6-diméthylphényl)-N-[(acétylthio) acétyl] alanine qui est le composé n° 1, dont les caractéristiques sont indiquées au tableau (I).

Exemple 4

Dans 70 $cm^3$ de méthanol on dissout 35 g d'ester méthylique de la N-(2,6-diméthylphényl)-N-[(acétylthio)-acétyl] alanine (composé n° 1 du tableau (I)).

A cette première solution, on ajoute progressivement en 1 heure, à 30 °C, une solution de méthylate de sodium préparée par adjonction de 2,5 g de sodium dans 150 $cm^3$ de méthanol.

Le milieu réactionnel issu du mélange de ces deux solutions, est acidifié avec 10 $cm^3$ d'une solution aqueuse d'acide chlorhydrique concentré. Le chlorure de sodium formé est filtré ; le filtrat est distillé sous vide de manière à en évaporer le méthanol ; le résidu issu de cette évaporation est dissous dans 50 $cm^3$ de chlorure de méthylène ; cette solution est lavée à l'eau jusqu'à neutralité des eaux de lavage puis séchée sur sulfate de sodium. On élimine alors le chlorure de méthylène par évaporation ; le résidu huileux est mélangé intimement avec 100 $cm^3$ d'hexane ce qui fournit un précipité cristallin constitué d'ester méthylique de la N-(2,6-diméthylphényl)-N-(mercaptoacétyl) alanine, qui est le composé n° 55 du tableau (II) ; ce tableau indique le point de fusion et l'analyse élémentaire de ce composé. Ce composé a été ainsi obtenu avec un rendement de 82 %.

Exemple 5

323 g (1 mole) d'ester méthylique de la N-(2,6-diméthylphényl)-N-[(acéthylthio) acétyl] alanine ou composé n° 1 du tableau (I), sont dissous dans un litre de méthanol. On ajoute 10 $cm^3$ de solution aqueuse concentrée d'acide chlorhydrique et chauffe pendant 5 h à l'ébullition à reflux. Le méthanol est éliminé par distillation. Le résidu huileux est cristallisé par agitation dans l'éther de pétrole. Le précipité est filtré, puis séché sous pression absolue réduite à 27 millibars. On obtient, avec un rendement de 98 %, l'ester méthylique de la N-(2,6-diméthylphényl)-N-(mercaptoacétyl) alanine, qui est le composé n° 55 du tableau (II).

Exemple 6

Dans 40 $cm^3$ de toluène on dissout 2,5 g de triéthylamine et 7 g d'ester méthylique de la N-(2,6-diméthylphényl)-N-(mercaptoacétyl) alanine de formule

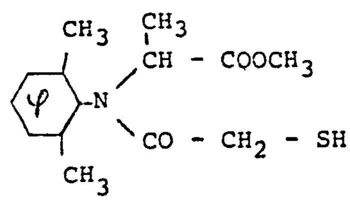

A cette solution maintenue sous agitation, on ajoute progressivement en 20 mn une solution de 3,6 g de chlorure de l'acide diméthyl-3,3 butanoïque dans 20 $cm^3$ de toluène ; la température augmente depuis une valeur de 20° C jusqu'à une valeur de 30 °C au cours de cette addition. On poursuit l'agitation pendant une demi-heure ; le chlorhydrate de triéthylamine est éliminé par essorage ; la solution toluénique restante, issue de cet essorage, est lavée avec une solution de bicarbonate de sodium puis à l'eau. Le toluène est éliminé sous pression réduite et l'on obtient alors une huile constituée par l'ester méthylique de la N-(2,6-diméthylphényl)-N-[(diméthyl-3,3 butanoylthio) acétyl] alanine de formule

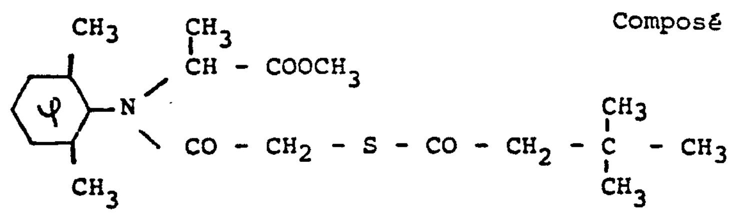

Composé n° 10

Ce produit a été obtenu avec un rendement de 84 %.
Le point de fusion et l'analyse élémentaire sont indiqués dans le tableau (I).

Exemple 7

Dans 100 $cm^3$ de chlorure de méthylène, on dissout :
— 14,05 g (0,05 mole) de N-(2,6-diméthylphényl)-N-(mercaptoacétyl) alaninate de méthyle de formule

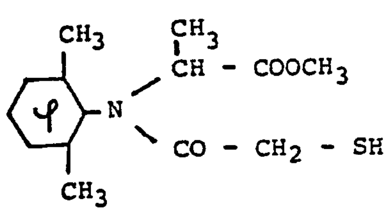

— 4,25 g (0,05 mole) d'acide cyanacétique

($N \equiv C—CH_2—COOH$).

On agite à 20 °C et ajoute progressivement en 15 mn une solution de 10,3 g de dicyclohexylcarbodiimide dans 50 $cm^3$ de chlorure de méthylène. On chauffe alors à l'ébullition à reflux pendant 2 h puis refroidit à 0 °C. On filtre le précipité de dicyclohexylurée, élimine le solvant par distillation, ajoute au résidu 50 $cm^3$ d'éther diéthylique et refroidit à −20°. Le précipité est filtré et séché. On obtient 9 g (rendement 51 %) de N-[(cyanacétylthio) acétyl]-N-(2,6-diméthylphényl) alaninate de méthyle (composé n° 48) dont le point de fusion et l'analyse élémentaire sont indiqués au tableau I.

Exemple 8

On chauffe pendant 2 h à l'ébullition à reflux une solution constituée de :
— 70,25 g (0,25 mole) de N-(2,6-diméthylphényl)-N-(mercaptoacétyl) alaninate de méthyle
— 200 $cm^3$ d'une solution aqueuse de soude 0,125 N.
On concentre à sec sous pression réduite.

On obtient ainsi 72,3 g (rendement : 100 %) de N-(2,6-diméthylphényl)-N-(mercaptoacétyl) alaninate de sodium (composé n° 75) dont le point de fusion et l'analyse élémentaire sont indiqués au tableau (III). La réaction effectuée a été

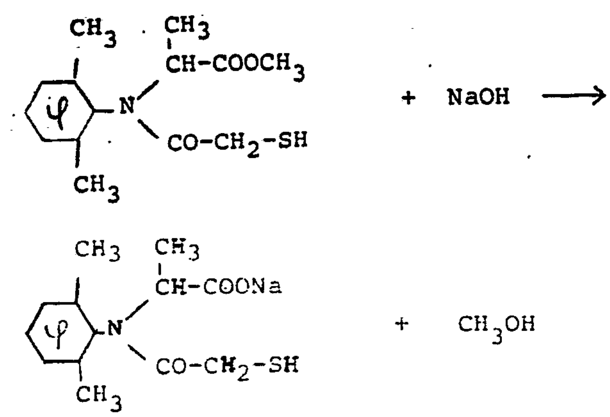


Exemple 9

On chauffe à l'ébullition à reflux jusqu'à dissolution (pendant 2 h) une suspension constituée de 50 $cm^3$ d'eau, 0,4 g (0,01 mole) de MgO et de 6,18 g (0,02 mole) de N-[(acétylthio) acétyl]-N-(2,6-diméthylphényl) alanine. On refroidit à 0 °C. Le précipité est filtré, essoré, puis séché à 40 °C sous pression absolue de 27 millibars et en présence de $P_2O_5$.

On obtient 4,6 g (rendement 72 %) de N-[(acétylthio) acétyl]-N-(2,6-diméthylphényl) alaninate de magnésium dont le point de fusion et l'analyse élémentaire sont indiqués au tableau (III). La réaction effectuée a été

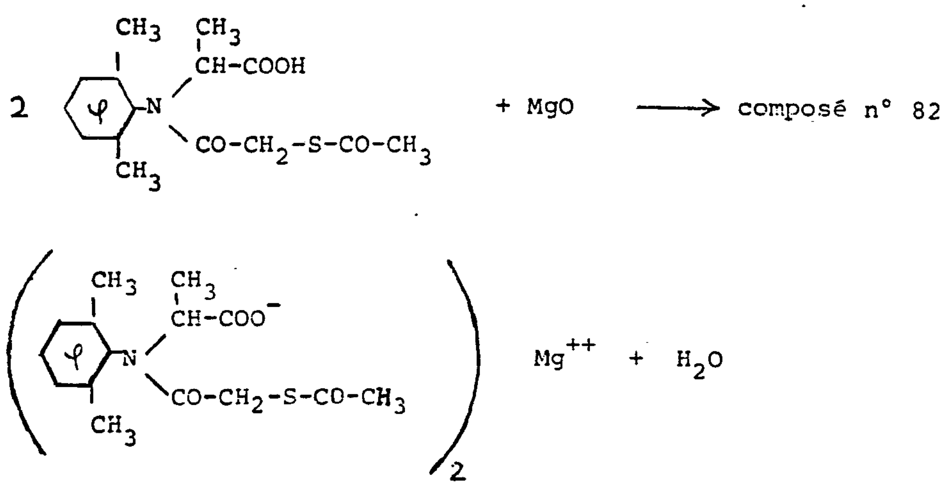

Exemple 10

Une solution de 2,5 g (0,01 mole) de Cu $SO_4$ dans 50 $cm^3$ d'eau est ajoutée progressivement en 15 mn et sous agitation à une solution constituée de 50 $cm^3$ d'eau et de 6,62 g (0,02 mole) de N-[(acétylthio) acétyl]-N-(2,6-diméthylphényl) alaninate de sodium. L'agitation est poursuivie encore 15 mn, le précipité est filtré, lavé à l'eau, essoré puis séché à 40 °C sous pression absolue réduite à 27 millibars en présence de $P_2O_5$.

On obtient ainsi 5,5 g (rendement 92 %) de N-[(acétylthio) acétyl]-N-(2,6-diméthylphényl) alaninate cuivrique (composé n° 84) dont le point de fusion et l'analyse élémentaire sont indiqués au tableau (III). La réaction effectuée a été

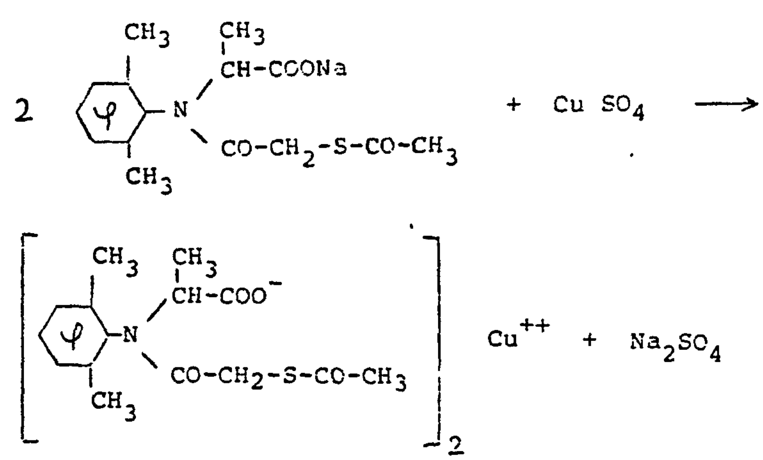

Les composés ci-après peuvent également être préparés d'une manière semblable à ceux décrits ci-avant :

N-(2,6-diméthylphényl)-N-[(propionylthio) acetyl] glycinate de méthyle
N-(3-chloro-2,6-diméthylphényl)-N-[(propionylthio) acetyl] glycinate de méthyle
N-(3-bromo-2,6-diméthylphényl)-N-[(propionylthio) acetyl] glycinate de méthyle
N-[(propionylthioacetyl]-N-(2,3,6-triméthylphényl) glycinate de méthyle
N-(3-chloro-2,6-diméthylphényl)-N-[(propionylthio) acetyl] alaninate de méthyle
N-(3-bromo-2,6-diméthylphényl)-N-[(propionylthio) acetyl] alaninate de méthyle
N-[(propionylthio) acetyl]-N-(2,3,6-triméthylphényl) alaninate de méthyle
N-[(butyrylthio) acetyl]-N-(2,6-diméthylphényl) glycinate de méthyle
N-[(butyrylthio) acetyl]-N-(3-chloro-2,6-diméthylphényl) glycinate de méthyle
N-(3-bromo-2,6-diméthylphényl)-N-[(butyrylthio) acétyl] glycérinate de méthyle
N-[(butyrylthio) acetyl]-N-(2,3,6-triméthylphényl) glycinate de méthyle
N-[(butyrylthio) acetyl]-N-(3-chloro-2,6-diméthylphényl) alaninate de méthyle
N-(3-bromo-2,6-diméthylphényl)-N-[(butyrylthio) acetyl] alaninate de méthyle
N-[(butyrylthio) acetyl]-N-(2,3,6-triméthylphényl) alaninate de méthyle
N-(2,6-diméthylphényl)-N-[(isobutyrylthio) acetyl] glycinate de méthyle
N-(3-chloro-2,6-diméthylphényl)-N-[(isobutyrylthio) acetyl] glycinate de méthyle
N-(3-bromo-2,6-diméthylphényl)-N-[(isobutyrylthio) acetyl] glycinate de méthyle
N-[(isobutyrylthio) acetyl]-N-(2,3,6-triméthylphényl) glycinate de méthyle
N-(3-chloro-2,6-diméthylphényl)-N-[(isobutyrylthio) acetyl] alaninate de méthyle
N-(3-bromo-2,6-diméthylphényl)-N-[(isobutyrylthio) acetyl] alaninate de méthyle
N-[(isobutyrylthio) acetyl]-N-(2,3,6-triméthylphényl) alaninate de méthyle
N-(2,6-diméthylphényl)-N-[(pentanoylthio) acetyl] glycinate de méthyle
N-(3-chloro-2,6-diméthylphényl)-N-[(pentonoylthio) acetyl] glycinate de méthyle
N-(3-bromo-2,6-diméthylphényl)-N-[(pentanoylthio) acetyl] glycinate de méthyle
N-[(pentanoylthio) acetyl]-N-(2,3,6-triméthylphényl) glycinate de méthyle
N-(3-chloro-2,6-diméthylphényl)-N-[(pentanoylthio) acetyl] alaninate de méthyle
N-(3-bromo-2,6-diméthylphényl)-N-[(pentanoylthio) acetyl] alaninate de méthyle
N-[(pentanoylthio) acetyl]-N-(2,3,6-triméthylphényl) alaninate de méthyle
N-(2,6-diméthylphényl)-N-[(3-méthylbutyrylthio) acetyl] glycinate de méthyle
N-(3-chloro-2,6-diméthylphényl)-N-[(3-méthylbutyrylthio) acetyl] glycinate de méthyle
N-(3-bromo-2,6-diméthylphényl)-N-[(3-méthylbutyrylthio) acetyl] glycinate de méthyle
N-(3-méthylbutyrylthio) acetyl]-N-(2,3,6-triméthylphényl) glycinate de méthyle
N-(3-chloro-2,6-diméthylphényl)-N-[(3-méthylbutyrylthio) acetyl] alaninate de méthyle
N-(3-bromo-2,6-diméthylphényl)-N-[(3-méthylbutyryltio) acetyl] alaninate de méthyle

N-(2,6-diméthylphényl)-N-[(2-méthylbutyrylthio) acetyl] glycinate de méthyle
N-(3-chloro-2,6-diméthylphényl)-N-[(2-méthylbutyrylthio) acetyl] glycinate de méthyle
N-(3-bromo-2,6-diméthylphényl)-N-[(2-méthylbutyrylthio) acetyl] glycinate de méthyle
N-[(2-méthylbutyrylthio) acetyl]-N-(2,3,6-triméthylphényl) glycinate de méthyle
N-(3-chloro-2,6-diméthylphényl)-N-[(2-méthylbutyrylthio) acetyl] alaninate de méthyle
N-(3-bromo-2,6-diméthylphényl)-N-[(2-méthylbutyrylthio) acetyl] alaninate de méthyle
N-[(2-méthylbutyrylthio) acetyl]-N-(2,3,6-triméthylphényl) alaninate de méthyle
N-[(3-méthylbutyrylthio) acetyl]-N-(2,3,6-triméthylphényl) alaninate de méthyle
N-(2,6-diméthylphényl)-N-[(2,2-diméthylpropionylthio) acetyl] glycinate de méthyle
N-(3-chloro-2,6-diméthylphényl)-N-[(2,2-diméthylpropionylthio) acetyl] glycinate de méthyle
N-(3-bromo-2,6-diméthylphényl)-N-[(2,2-diméthylpropionylthio) acetyl] glycinate de méthyle
N-[(2,2-diméthylpropionylthio) acetyl]-N-(2,3,6-triméthylphényl) glycinate de méthyle
N-(3-chloro-2,6-diméthylphényl)-N-[(2,2-diméthylpropionylthio) acetyl] alaninate de méthyle
N-(3-bromo-2,6-diméthylphényl)-N-[(2,2-diméthylpropionylthio) acetyl] alaninate de méthyle
N-[(2,2-diméthylpropionylthio) acetyl]-N-(2,3,6-triméthylphényl) alaninate de de méthyle
N-(2,6-diméthylphényl)-N-[(hexanoylthio) acetyl] glycinate de méthyle
N-(3-chloro-2,6-diméthylphényl)-N-[(hexanoylthio) acetyl] glycinate de méthyle
N-(3-bromo-2,6-diméthylphényl)-N-[(hexanoylthio) acetyl] glycinate de méthyle
N-[(hexanoylthio) acetyl]-N-(2,3,6-triméthylphényl) glycinate de méthyle
N-(3-chloro-2,6-diméthylphényl)-N-[(hexanoylthio) acetyl] alaninate de méthyle
N-(3-bromo-2,6-diméthylphényl)-N-[(hexanoylthio) acetyl] alaninate de méthyle
N-[(hexanoylthio) acetyl]-N-(2,3,6-triméthylphényl) alaninate de méthyle
N-[(3,3-diméthylbutyrylthio) acetyl]-N-(2,6-diméthylphényl) glycinate de méthyle
N-(3-chloro-2,6-diméthylphényl)-N-[(3,3-diméthylbutyrylthio) acetyl] glycinate de méthyle
N-(3-bromo-2,6-diméthylphényl)-N-[(3,3-diméthylbutyrylthio) acetyl] glycinate de méthyle
N-[(3,3-diméthylbutyrylthio) acetyl]-N-(2,3,6-triméthylphényl) glycinate de méthyle
N-(3-chloro-2,6-diméthylphényl)-N-[(3,3-diméthylbutyrylthio) acetyl] alaninate de méthyle
N-(3-bromo-2,6-diméthylphényl)-N-[(3,3-diméthylbutyrylthio) acetyl] alaninate de méthyle
N-[(3,3-diméthylbutylthio) acetyl]-N-(2,3,6-triméthylphényl) alaninate de méthyle
N-(2,6-diméthylphényl)-N-[(2-éthylbutyrylthio) acetyl] glycinate de méthyle
N-(3-chloro-2,6-diméthylphényl)-N-[(2-éthylbutyrylthio) acetyl] glycinate de méthyle
N-(3-bromo-2,6-diméthylphényl)-N-[(2-éthylbutyrylthio) acetyl] glycinate de méthyle
N-[(2-éthylbutyrylthio) acetyl]-N-(2,3,6-triméthylphényl) glycinate de méthyle
N-(3-chloro-2,6-diméthylphényl)-N-[(2-éthylbutyrylthio) acetyl] alaninate de méthyle
N-(3-bromo-2,6-diméthylphényl)-N-[(2-éthylbutyrylthio) acetyl] alaninate de méthyle
N-[(2-éthylbutyrylthio) acetyl]-N-(2,3,6-triméthylphényl) alaninate de méthyle
N-(2,6-diméthylphényl)-N-[(heptanoylthio) acetyl] glycinate de méthyle
N-(3-chloro-2,6-diméthylphényl)-N-[(heptanoylthio) acetyl] glycinate de méthyle
N-(3-bromo-2,6-diméthylphényl)-N-[(heptanoylthio) acetyl] glycinate de méthyle
N-[(heptanoylthio) acetyl]-N-(2,3,6-triméthylphényl) glycinate de méthyle
N-(3-chloro-2,6-diméthylphényl)-N-[(heptanoylthio) acetyl] alaninate de méthyle
N-(3-bromo-2,6-diméthylphényl)-N-[(heptanoylthio) acetyl] alaninate de méthyle
N-[(heptanoylthio) acetyl]-N-(2,3,6-triméthylphényl) alaninate de méthyle
N-(2,6-diméthylphényl)-N-[(2-éthylhexanoylthio) acetyl] glycinate de méthyle
N-(3-chloro-2,6-diméthylphényl)-N-[(2-éthylhexanoylthio) acetyl] glycinate de méthyle
N-(3-bromo-2,6-diméthylphényl)-N-[(2-éthylhexanoylthio) acetyl] glycinate de méthyle
N-[(2-éthylhexanoylthio) acetyl]-N-(2,3,6-trimethylphényl) glycinate de méthyle
N-(3-chloro-2,6-diméthylphényl)-N-[(2-éthylhexanoylthio) acetyl] alaninate de méthyle
N-(3-bromo-2,6-diméthylphényl)-N-[(2-éthylhexanoylthio) acetyl] alaninate de méthyle
N-[(2-éthylhexanoylthio) acetyl]-N-(2,3,6-triméthylphényl) alaninate de méthyle
N-(2,6-diméthylphényl)-N-[(octanoylthio) acetyl] glycinate de méthyle
N-(3-chloro-2,6-diméthylphényl)-N-[(octanoylthio) acetyl] glycinate de méthyle
N-(3-bromo-2,6-diméthylphényl)-N-[(octanoylthio) acetyl] glycinate de méthyle
N-[(octanoylthio) acetyl]-N-(2,3,6-triméthylphényl) glycinate de méthyle
N-(3-chloro-2,6-diméthylphényl)-N-[(octanoylthio) acetyl] alaninate de méthyle
N-(3-bromo-2,6-diméthylphényl)-N-[(octanoylthio) acetyl] alaninate de méthyle
N-[(octanoylthio) acetyl]-N-(2,3,6-triméthylphényl) alaninate de méthyle
N-(2,6-diméthylphényl)-N-[(nonanoylthio) acetyl] glycinate de méthyle
N-(3-chloro-2,6-diméthylphényl)-N-[(nonanoylthio) acetyl] glycinate de méthyle
N-(3-bromo-2,6-diméthylphényl)-N-[(nonanoylthio) acetyl] glycinate de méthyle
N-[(nonanoylthio) acetyl]-N-(2,3,6-triméthylphényl) glycinate de méthyle
N-(3-chloro-2,6-diméthylphényl)-N-[(nonanoylthio) acetyl] alaninate de méthyle
N-(3-bromo-2,6-diméthylphényl)-N-[(nonanoylthio) acetyl] alaninate de méthyle
N-[(nonanoylthio) acetyl]-N-(2,3,6-triméthylphényl) alaninate de méthyle
N-(2,6-diméthylphényl)-N-[(décanoylthio) acetyl] glycinate de méthyle

N-(3-chloro-2,6-diméthylphényl)-N-[(décanoylthio) acetyl] glycinate de méthyle
N-(3-bromo-2,6-diméthylphényl)-N-[(décanoylthio) acetyl] glycinate de méthyle
N-[(décanoylthio) acetyl]-N-(2,3,6-triméthylphényl) glycinate de méthyle
N-(3-chloro-2,6-diméthylphényl)-N-[(décanoylthio) acetyl] alaninate de méthyle
N-(3-bromo-2,6-diméthylphényl)-N-[(décanoylthio) acetyl] alaninate de méthyle
N-[(décanoylthio) acetyl]-N-(2,3,6-triméthylphényl) alaninate de méthyle
N-(2,6-diméthylphényl)-N-[(undécanoylthio) acetyl] glycinate de méthyle
N-(3-chloro-2,6-diméthylphényl)-N-[(undécanoylthio) acetyl] glycinate de méthyle
N-(3-bromo-2,6-diméthylphényl)-N-[(undécanoylthio) acetyl] glycinate de méthyle
N-(2,3,6-triméthylphényl)-N-[(undécanoylthio) acetyl] glycinate de méthyle
N-(3-chloro-2,6-diméthylphényl)-N-[(undécanoylthio) acetyl] alaninate de méthyle
N-(3-bromo-2,6-diméthylphényl)-N-[(undécanoylthio) acetyl] alaninate de méthyle
N-(2,3,6-triméthylphényl)-N-[(undécanoylthio) acetyl] alaninate de méthyle
N-(2,6-diméthylphényl)-N-[(dodécanoylthio) acetyl] glycinate de méthyle
N-(3-chloro-2,6-diméthylphényl)-N-[(dodécanoylthio) acetyl] glycinate de méthyle
N-(3-bromo-2,6-diméthylphényl)-N-[(dodécanoylthio) acetyl] glycinate de méthyle
N-[(dodécanoylthio) acetyl]-N-(2,3,6-triméthylphényl) glycinate de méthyle
N-(3-chloro-2,6-diméthylphényl)-N-[(dodécanoylthio) acetyl] alaninate de méthyle
N-(3-bromo-2,6-diméthylphényl)-N-[(dodécanoylthio) acetyl] alaninate de méthyle
N-[(dodécanoylthio) acetyl]-N-(2,3,6-triméthylphényl) alaninate de méthyle
N-(2,6-diméthylphényl)-N-[(tetradécanoylthio) acetyl] glycinate de méthyle
N-(3-chloro-2,6-diméthylphényl)-N-[(tetradécanoylthio) acetyl] glycinate de méthyle
N-(3-bromo-2,6-diméthylphényl)-N-[(tetradécanoylthio) acetyl] glycinate de méthyle
N-[(tetradécanoylthio) acetyl]-N-(2,3,6-triméthylphényl) glycinate de méthyle
N-(3-chloro-2,6-diméthylphényl)-N-[(tetradécanoylthio) acetyl] alaninate de méthyle
N-(3-bromo-2,6-diméthylphényl)-N-[(tetradécanoylthio) acetyl] alaninate de méthyle
N-[(tetradécanoylthio) acetyl]-N-(2,3,6-triméthylphényl) alaninate de méthyle
N-(2,6-diméthylphényl)-N-[(hexadécanoylthio) acetyl] glycinate de méthyle
N-(3-chloro-2,6-diméthylphényl)-N-[(hexadécanoylthio) acetyl] glycinate de méthyle
N-(3-bromo-2,6-diméthylphényl)-N-[(hexadécanoylthio) acetyl] glycinate de méthyle
N-[(hexadécanoylthio) acetyl]-N-(2,3,6-triméthylphényl) glycinate de méthyle
N-(3-chloro-2,6-diméthylphényl)-N-[(hexadécanoylthio) acetyl] alaninate de méthyle
N-(3-bromo-2,6-diméthylphényl)-N-[(hexadécanoylthio) acetyl] alaninate de méthyle
N-[(hexadécanoylthio) acetyl]-N-(2,3,6-triméthylphényl) alaninate de méthyle
N-(2,6-diméthylphényl)-N-[(octadécanoylthio) acetyl] glycinate de méthyle
N-(3-chloro-2,6-diméthylphényl)-N-[(octadécanoylthio) acetyl] glycinate de méthyle
N-(3-bromo-2,6-diméthylphényl)-N([(octadécanoylthio) acetyl] glycinate de méthyle
N-[(octadécanoylthio) acetyl]-N-(2,3,6-triméthylphényl) glycinate de méthyle
N-(3-chloro-2,6-diméthylphényl)-N-[(octadécanoylthio) acetyl] alaninate de méthyle
N-(3-bromo-2,6-diméthylphényl)-N-[(octadécanoylthio) acetyl] alaninate de méthyle
N-[(octadécanoylthio) acetyl]-N-(2,3,6-triméthylphényl) alaninate de méthyle
N-[(cyclohexanecarbonylthio) acetyl]-N-(2,6-diméthylphényl) glycinate de méthyle
N-(3-chloro-2,6-diméthylphényl)-N-[(cyclohexanecarbonylthio) acetyl] glycinate de méthyle
N-(3-bromo-2,6-diméthylphényl)-N-[(cyclohexanecarbonylthio) acetyl] glycinate de méthyle
N-[(cyclohexanecarbonylthio) acetyl]-N-(2,3,6-triméthylphényl) glycinate de méthyle
N-(3-chloro-2,6-diméthylphényl)-N-[(cyclohexylcarbonylthio) acetyl] alaninate de méthyle
N-(3-bromo-2,6-diméthylphényl)-N-[(cyclohexylcarbonylthio) acetyl] alaninate de méthyle
N-[(cyclohexylcarbonylthio) acetyl]-N-(2,3,6-triméthylphényl) alaninate de méthyle
N-[(cyclopentylcarbonylthio) acetyl]-N-(2,6-diméthylphényl) glycinate de méthyle
N-(3-chloro-2,6-diméthylphényl)-N-[(cyclopentylcarbonylthio) acetyl] glycinate de méthyle
N-(3-bromo-2,6-diméthylphényl)-N-[(cyclopentylcarbonylthio) acetyl] glycinate de méthyle
N-[(cyclopentylcarbonylthio) acetyl]-N-(2,3,6-triméthylphényl) glycinate de méthyle
N-(3-chloro-2,6-diméthylphényl)-N-[(cyclopentylcarbonylthio) acetyl] alaninate de méthyle
N-(3-bromo-2,6-diméthylphényl)-N-[(cyclopentylcarbonylthio) acetyl] alaninate de méthyle
N-[(cyclopentylcarbonylthio) acetyl]-N-(2,3,6-triméthylphényl) alaninate de méthyle
N-[(3,3-diméthylacryloylthio) acetyl]-N-(2,6-diméthylphényl) glycinate de méthyle
N-(3-chloro-2,6-diméthylphényl)-N-[(3,3-diméthylacryloylthio) acetyl] glycinate de méthyle
N-(3-bromo-2,6-diméthylphényl)-N-[(3,3-diméthylacryloylthio) acetyl] glycinate de méthyle
N-[(3,3-diméthylacryloylthio) acetyl]-N-(2,3,6-triméthylphényl) glycinate de méthyle
N-(3-chloro-2,6-diméthylphényl)-N-[(3,3-diméthylacryloylthio) acetyl] alaninate de méthyle
N-(3-bromo-2,6-diméthylphényl)-N-[(3,3-diméthylacryloylthio) acetyl] alaninate de méthyle
N-[(3,3-diméthylacryloylthio) acetyl]-N-(2,3,6-triméthylphényl) alaninate de méthyle
N-[(benzoylthio) acetyl]-N-(2,6-diméthylphényl) glycinate de méthyle
N-[(benzoylthio) acetyl]-N-(3-chloro-2,6-diméthylphényl) glycinate de méthyle
N-[(benzoylthio) acetyl]-N-(3-bromo-2,6-diméthylphényl) glycinate de méthyle

N-[(benzoylthio) acetyl]-N-(2,3,6-triméthylphényl) glycinate de méthyle
N-[(benzoylthio) acetyl]-N-(3-chloro-2,6-diméthylphényl) alaninate de méthyle
N-[(benzoylthio) acetyl]-N-(3-bromo-2,6-diméthylphényl) alaninate de méthyle
N-[(benzoylthio] acetyl]-N-(2,3,6-triméthylphényl) alaninate de méthyle
N-(2,6-diméthylphényl)-N-[(methoxyacétylthio) acetyl] glycinate de méthyle
N-(3-chloro-2,6-diméthylphényl)-N-[(methoxyacéthylthio) acetyl] glycinate de méthyle
N-(3-bromo-2,6-diméthylphényl)-N-[(methoxyacétylthio) acetyl] glycinate de méthyle
N-[(methoxyacétylthio) acetyl]-N-(2,3,6-triméthylphényl) glycinate de méthyle
N-(3-chloro-2,6-diméthylphényl)-N-[(méthoxyacétylthio) acetyl] alaninate de méthyle
N-(3-bromo-2,6-diméthylphényl)-N-[(methoxyacétylthio) acetyl] alaninate de méthyle
N-[(methoxyacétylhio) acetyl]-N-(2,3,6-triméthylphényl) alaninate de méthyle
N-(2,6-diméthylphényl)-N-[(2-furanylcarbonylthio) acetyl] glycinate de méthyle
N-(3-chloro-2,6-diméthylphényl)-N-[(2-furanylcarbonylthio) acetyl] glycinate de méthyle
N-(3-bromo-2,6-diméthylphényl)-N-[(2-furanylcarbonylthiuo) acetyl] glycinate de méthyle
N-(2-furanylcarbonylthio) acetyl]-N-(2,3,6-triméthylphényl) glycinate de méthyle
N-(3-chloro-2,6-diméthylphényl)-N-[(2-furanylcarbonylthio) acetyl] alaninate de méthyle
N-(3-bromo-2,6-diméthylphényl)-N-[(2-furanylcarbonylthio) acetyl] alaninate de méthyle
N-[(2-furanylcarbonylthio) acetyl]-N-(2,3,6-triméthylphényl) alaninate de méthyle
N-(2,6-diméthylphényl)-N-[(2-thienylcarbonylthio) acetyl] glycinate de méthyle
N-(3-chloro-2,6-diméthylphényl)-N-[(2-thienylcarbonylthio) acetyl] glycinate de méthyle
N-(3-bromo-2,6-diméthylphényl)-N-[(2-thienylcarbonylthio) acetyl] glycinate de méthyle
N-[(2-thienylcarbonylthio) acetyl]-N-(2,3,6-triméthylphényl) glycinate de méthyle
N-(3-chloro-2,6-diméthylphényl)-N-[2-thienylcarbonylthio) acetyl] alaninate de méthyle
N-(3-bromo-2,6-diméthylphényl)-N-[(2-thienylcarbonylthio) acetyl] alaninate de méthyle
N-[(2-thienylcarbonylthio) acetyl]-N-(2,3,6-triméthylphényl) alaninate de méthyle
N-[(acetylthio) acetyl]-N-(3-chloro-2,6-diméthylphényl) glycinate de méthyle
N-[(acetylthio) acetyl]-N-(3-bromo-2,6-diméthylphényl) glycinate de méthyle
N-[(acetylthio) acetyl]-N-(2,3,6-triméthylphényl) glycinate de méthyle
N-[(acetylthio) acetyl]-N-(3-bromo-2,6-diméthylphényl) alaninate de méthyle
N-[(acetylthio) acetyl]-N-(2,3,6-triméthylphényl) alaninate de méthyle
N-(3-chloro-2,6-diméthylphényl)-N-(mercaptoacetyl) glycinate de méthyle
N-(3-bromo-2,6-diméthylphényl)-N-(mercaptoacetyl) glycinate de méthyle
N-(mercaptoacetyl)-N-(2,3,6-triméthylphényl) glycinate de méthyle
N-(3-chloro-2,6-diméthylphényl)-N-(mercaptoacetyl) alaninate de méthyle
N-(3-bromo-2,6-diméthylphényl)-N-(mercaptoacetyl) alaninate de méthyle
N-(mercaptoacétyl)-N-(2,3,6-triméthylphényl) alaninate de méthyle

(Voir Tableaux I, II, et III, pages suivantes)

Tableau 1

Composés de formule

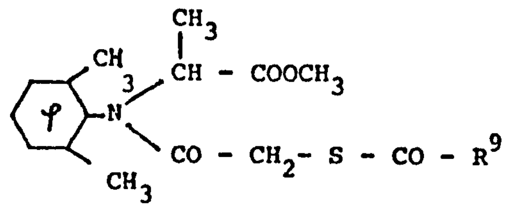

| Composé n° | $R^9$ | Caractéristiques physiques | | Analyse élémentaire | | | | | | | |
|---|---|---|---|---|---|---|---|---|---|---|---|
| | | Point de fusion en °C | $n_D^{20}$ | Valeurs calculées en % | | | | Valeurs trouvées en % | | | |
| | | | | C | H | N | S | C | H | N | S |
| 1 | $CH_3-$ | 52 | | 59,42 | 6,54 | 4,33 | | 59,48 | 6,60 | 4,23 | |
| 2 | $C_2H_5-$ | 57 | | | | | | | | | |
| 3 | $n-C_3H_7-$ | | 1,5335 | | | | | | | | |
| 4 | $(CH_3)_2CH-$ | | 1,5332 | 61,54 | 7,12 | 3,99 | 9,12 | 61,60 | 7,16 | 3,97 | 9,13 |
| 5 | $n-C_4H_9-$ | | 1,531 | 62,47 | 7,40 | 3,84 | 8,77 | 62,53 | 7,52 | 3,90 | 8,53 |
| 6 | $(CH_3)_2CH-CH_2-$ | | 1,529 | 62,44 | 7,45 | 3,8 | 8,77 | 62,9 | 7,40 | 3,53 | 8,67 |
| 7 | $CH_3-CH-$<br>$C_2H_5$ | | 1,5400 | 62,44 | 7,45 | 3,83 | | 61,46 | 7,30 | 3,97 | |

Tableau 1 (suite)

Composés de formule

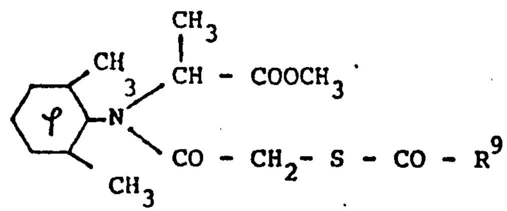

| Composé n° | $R^9$ | Caractéristiques physiques: Point de fusion en °C | Caractéristiques physiques: $n_D^{20}$ | Analyse élémentaire: Valeurs calculées en %: C | H | N | S | Valeurs trouvées en %: C | H | N | S |
|---|---|---|---|---|---|---|---|---|---|---|---|
| 8 | $(CH_3)_3C-$ | | 1,5299 | 62,47 | 7,39 | 3,84 | 8,77 | 62,55 | 7,39 | 3,74 | 8,71 |
| 9 | $n-C_5H_{11}-$ | | 1,526 | 63,30 | 7,70 | 3,69 | 8,45 | 63,35 | 7,58 | 3,71 | 8,15 |
| 10 | $(CH_3)_3C-CH_2-$ | 68 | | 63,22 | 7,65 | 3,69 | 8,44 | 63,38 | 7,58 | 3,67 | 8,36 |
| 11 | $(C_2H_5)_2-CH-$ | | 1,518 | 63,32 | 7,65 | 3,69 | 8,44 | 63,81 | 8,11 | 3,51 | 7,05 |
| 12 | $n-C_6H_{13}-$ | | 1,525 | 64,12 | 7,89 | 3,56 | 8,14 | 63,44 | 7,52 | 3,60 | 8,28 |
| 13 | $n-C_7H_{15}-$ | | 1,514 | 64,86 | 8,11 | 3,44 | 7,86 | 64,52 | 8,17 | 3,27 | 6,66 |
| 14 | $n-C_4H_9-CH(C_2H_5)-$ | | 1,5175 | 65,51 | 8,19 | 3,47 | 7,94 | 64,10 | 7,94 | 3,40 | 8,06 |
| 15 | $n-C_8H_{17}-$ | | 1,514 | | | | | | | | |

Tableau 1 (suite)

Composés de formule

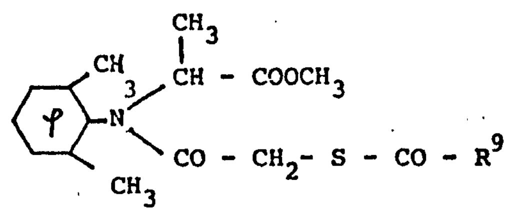

| Composé n° | $R^9$ | Caractéristiques physiques: Point de fusion en °C | Caractéristiques physiques: $n_D^{20}$ | Analyse élémentaire – Valeurs calculées en %: C | H | N | S | Analyse élémentaire – Valeurs trouvées en %: C | H | N | S |
|---|---|---|---|---|---|---|---|---|---|---|---|
| 16 | $n-C_9H_{19}-$ | | | | | | | | | | |
| 17 | $n-C_{10}H_{21}-$ | | 1,509 | 66,82 | 8,7 | 3,12 | 7,13 | 67,58 | 9,3 | 3,75 | 6,1 |
| 18 | $n-C_{11}H_{23}-$ | 35 | | 67,35 | 8,91 | 3,02 | 6,92 | 67,75 | 9,25 | 2,90 | 6,87 |
| 19 | $n-C_{13}H_{27}-$ | 37,5 | | 68,43 | 9,16 | 2,85 | 6,52 | 68,33 | 9,27 | 2,90 | 6,66 |
| 20 | $n-C_{16}H_{33}-$ | | | | | | | | | | |
| 21 | $n-C_{17}H_{35}-$ | 38 | | 70,20 | 9,67 | 2,56 | 5,85 | 69,78 | 9,73 | 3,07 | 5,04 |
| 22 | 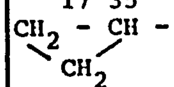 | 107 | | | | | | | | | |
| 23 | 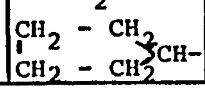 | 45 | | | | | | | | | |

Tableau 1 (suite)

Composés de formule

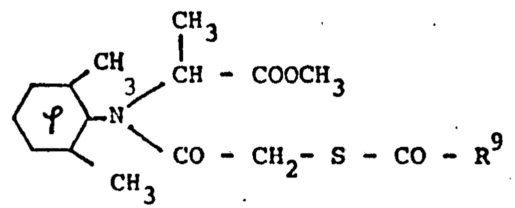

| Composé n° | $R^9$ | Caractéristiques physiques: Point de fusion en °C | Caractéristiques physiques: $n_D^{20}$ | Analyse élémentaire: Valeurs calculées en %: C | H | N | S | Valeurs trouvées en %: C | H | N | S |
|---|---|---|---|---|---|---|---|---|---|---|---|
| 24 | 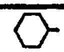 | 90,5 | | 64,42 | 7,47 | 3,58 | 8,19 | 64,10 | 7,63 | 3,55 | 8,31 |
| 25 | $(CH_3)_2C=CH-$ | 86 | | 62,79 | 6,93 | 3,85 | 8,82 | 62,67 | 6,91 | 3,67 | 8,97 |
| 26 | $C_6H_5-CH=CH-$ | 150,5 | | 67,13 | 6,12 | 3,40 | 7,79 | 66,92 | 6,34 | 3,36 | 7,78 |
| 27 | $CH_2=CH-(CH_2)_7-$ | | 1,515 | 67,11 | 8,28 | 3,13 | 7,16 | 66,50 | 8,20 | 3,00 | 7,50 |
| 28 | $CH_3(CH_2)_7-CH=CH-(CH_2)_7-$ | | 1,515 | 70,42 | 9,42 | 2,57 | 5,87 | 68,9 | 9,18 | 2,48 | 5,80 |
| 29 | 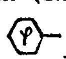 | 95 | | 65,43 | 6,01 | 3,63 | 8,32 | 65,32 | 6,14 | 3,56 | 8,75 |
| 30 | 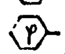 $CH_2-$ | 76,3 | | 66,17 | 6,27 | 3,51 | 8,02 | 65,95 | 6,28 | 3,51 | 8,36 |
| 31 | 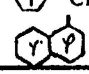 | 152,3 | | 68,97 | 5,75 | 3,22 | 7,36 | 68,75 | 5,82 | 3,19 | 7,40 |

Tableau 1 (suite)

Composés de formule

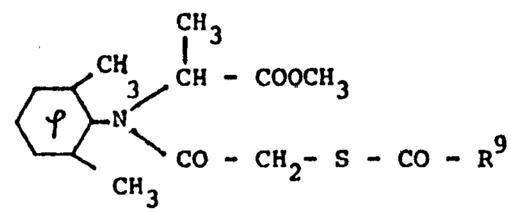

| Composé n° | $R^9$ | Caractéristiques physiques | | Analyse élémentaire | | | | | | | |
|---|---|---|---|---|---|---|---|---|---|---|---|
| | | Point de fusion en °C | $n_D^{20}$ | Valeurs calculées en % | | | | Valeurs trouvées en % | | | |
| | | | | C | H | N | S | C | H | N | S |
| 32 | 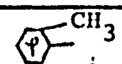 | 79,2 | | 66,17 | 6,27 | 3,51 | 8,02 | 65,98 | 6,37 | 3,45 | 8,00 |
| 33 | 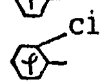 | 90,1 | | 60,07 | 5,24 | 3,34 | 7,63 | 60,03 | 5,26 | 3,31 | 7,70 |
| 34 | 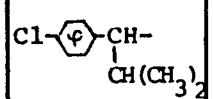 | 75 | | | | | | | | | |
| 35 | $Cl-CH_2-$ | | 1,5553 | 53,71 | 5,59 | 3,92 | 8,95 | 55,07 | 5,72 | 3,95 | 8,95 |
| 36 | $Cl_2\ CH-$ | 79 | | 48,98 | 4,85 | 3,57 | 8,16 | 50,03 | 5,11 | 3,68 | 8,33 |
| 37 | $Cl_3C-$ | | 1,555 | | | | | | | | |
| 38 | $CH_3O-CH_2-$ | 68,6 | | 57,79 | 6,52 | 3,97 | 9,06 | 58,18 | 6,50 | 3,97 | 9,73 |

Tableau 1 (suite)

Composés de formule

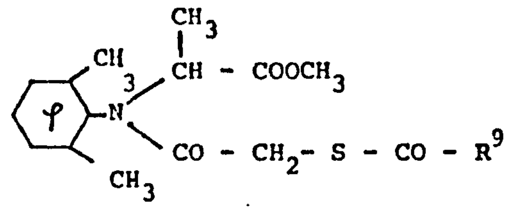

| Composé n° | $R^9$ | Caractéristiques physiques: Point de fusion en °C | Caractéristiques physiques: $n_D^{20}$ | Analyse élémentaire – Valeurs calculées en %: C | H | N | S | Valeurs trouvées en %: C | H | N | S |
|---|---|---|---|---|---|---|---|---|---|---|---|
| 39 | $CH_3-CHCl-$ | 53 | | 54,91 | 5,92 | 3,77 | 8,61 | 55,18 | 6,38 | 3,29 | 7,28 |
| 40 | $Cl-(CH_2)_3-$ | | 1,541 | 56,03 | 6,23 | 3,63 | 8,3 | 54,03 | 6,04 | 3,47 | 8,31 |
| 41 | $CH_3-S-CH_2-$ | | 1,562 | | | | | | | | |
| 42 | 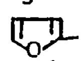 | 95 | | 60,78 | 5,64 | 3,73 | 8,54 | 60,92 | 5,59 | 3,77 | 8,64 |
| 43 | 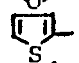 | 112 | | 58,29 | 5,41 | 3,58 | | 58,38 | 5,34 | 3,78 | |
| 44 | 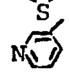 | 108 | | 62,3 | 5,7 | 7,3 | 8,2 | 61,8 | 6,9 | 6,9 | 8,66 |
| 45 | 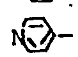 | 103 | | | | | | | | | |
| 46 | 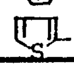 $CH_2-$ | | | | | | | | | | |

Tableau 1 (suite)

Composés de formule

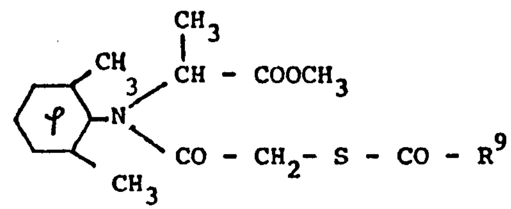

| Composé n° | $R^9$ | Caractéristiques physiques | | Analyse élémentaire | | | | | | | |
|---|---|---|---|---|---|---|---|---|---|---|---|
| | | Point de fusion en °C | $n_D^{20}$ | Valeurs calculées en % | | | | Valeurs trouvées en % | | | |
| | | | | C | H | N | S | C | H | N | S |
| 47 | 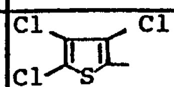 | 156,5 | | 46,15 | 3,64 | 2,9 | 13,3 | 46,25 | 3,56 | 2,7 | 13,30 |
| 48 | $N{\equiv}C{-}CH_2{-}$ | 128 | | 58,62 | 5,75 | 8,05 | | 58,83 | 5,90 | 7,90 | |
| 49 | $C_2H_5{-}O{-}CO{-}CH_2{-}$ | 66 | | 57,72 | 6,32 | 3,54 | | 57,69 | 6,44 | 3,56 | |
| 50 | $CH_3{-}CO{-}CH_2{-}$ | 78 | | 59,18 | 6,30 | 3,83 | | 59,04 | 6,26 | 3,88 | |
| 51 | $C_2H_5{-}O{-}CO{-}$ | 55,4 | | 56,69 | 6,03 | 3,67 | | 56,75 | 6,08 | 3,76 | |
| 52 | $C_2H_5{-}O{-}CO{-}CH{=}CH{-}$ (trans) | 70,3 | | 58,97 | 6,14 | 3,44 | | 58,97 | 6,33 | 3,41 | |
| 53 | $CH_3{-}CO{-}NH{-}CH_2{-}$ | 133 | | 56,84 | 6,31 | 7,36 | | 54,9 | 6,35 | 7,20 | |
| 54 | $CH_3{-}CO{-}O{-}$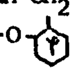 | 112 | | | | | | | | | |

Tableau II

Composés de formule

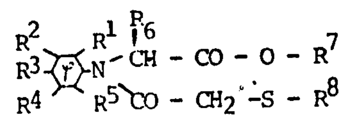

| Composé n° | $R^1$- | $R^2$- | $R^3$- | $R^4$- | $R^5$- | $R^6$- | $R^7$- | $R^8$- | Caractéristiques physiques: Point de fusion en °C | $n_D^{20}$ | analyse élémentaire 1° ligne : valeurs calculées en % 2° ligne : valeurs trouvées en % — C | H | N | S |
|---|---|---|---|---|---|---|---|---|---|---|---|---|---|---|
| 55 | $CH_3$- | H- | H- | H- | $CH_3$- | $CH_3$- | $CH_3$- | H- | 64 | | 59,76 | 6,81 | 4,98 | 11,37 |
| | | | | | | | | | | | 59,76 | 6,75 | 4,63 | 11,35 |
| 56 | $CH_3$- | Cl- | $CH_3$- | H- | $CH_3$- | $CH_3$- | $CH_3$- | $CH_3$- CO- | | 1,553 | 54,91 | 5,96 | 3,77 | 8,62 |
| | | | | | | | | | | | 54,70 | 6,11 | 3,60 | 8,70 |
| 57 | Cl- | H- | $CH_3$- | H- | H- | $CH_3$- | $CH_3$- | $CH_3$- CO- | | 1,547 | 52,40 | 5,28 | 4,07 | 9,33 |
| | | | | | | | | | | | 52,80 | 5,50 | 4,10 | 8,80 |
| 58 | $(CH_3)_2CH$- | H- | H- | H- | H- | $CH_3$- | $CH_3$- | $CH_3$- CO- | | 1,535 | | | | |
| 59 | $C_2H_5$- | H- | H- | H- | $C_2H_5$- | $CH_3$- | $CH_3$- | $CH_3$- CO- | | 1,537 | 61,51 | 7,17 | 3,99 | 9,12 |
| | | | | | | | | | | | 61,75 | 7,35 | 3,95 | 9,0 |
| 60 | $CH_3O$ - | H- | H- | H- | H- | $CH_3$- | $CH_3$- | $CH_3$- CO- | 48,5 | | 55,37 | 5,89 | 4,30 | 9,85 |
| | | | | | | | | | | | 55,21 | 6,02 | 4,28 | 9,75 |
| 61 | Br- | H- | H- | H- | H- | $CH_3$- | $CH_3$- | $CH_3$- CO- | 93 | | 44,93 | 4,31 | 3,74 | 8,57 |
| | | | | | | | | | | | 44,94 | 4,38 | 3,67 | 8,32 |

Tableau II (suite)

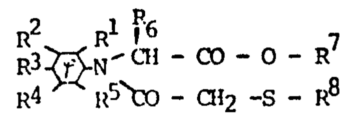

| Composé n° | R¹- | R²- | R³- | R⁴- | R⁵- | R⁶- | R⁷- | R⁸- | Point de fusion en °C | $n_D^{20}$ | C | H | N | S |
|---|---|---|---|---|---|---|---|---|---|---|---|---|---|---|
| | | | | | | | | | | | analyse élémentaire 1°ligne : valeurs calculées 2°ligne : valeurs trouvées | | | |
| 62 | $CH_3$- | Br- | $CH_3$- | H- | $CH_3$- | $CH_3$ | $CH_3$- | $CH_3$ CO - | | | 49,04 | 5,29 | 3,37 | 7,69 |
| | | | | | | | | | | | 49,15 | 5,55 | 3,20 | 7,91 |
| 63 | H- | Cl- | H- | H- | $CH_3O$ - | $CH_3$- | $CH_3$- | $CH_3$ CO— | 68,5 | | 50,07 | 5,04 | 3,89 | 8,91 |
| | | | | | | | | | | | 50,17 | 5,17 | 3,82 | 8,86 |
| 64 | $CH_3$ - | H- | $CH_3$- | H- | $CH_3$- | $CH_3$- | $CH_3$- | $CH_3$ CO - | | 1,541 | 60,90 | 6,87 | 4,15 | 9,50 |
| | | | | | | | | | | | 60,40 | 6,90 | 4,08 | 9,40 |
| 65 | Cl- | H- | H- | H- | $CH_3$- | $CH_3$- | $CH_3$- | $CH_3$ CO - | | 1,548 | | | | |
| 66 | $CH_3$- | Cl- | H- | H- | $CH_3$- | $CH_3$- | $CH_3$- | $CH_3$ CO - | | 1,551 | 53,80 | 5,60 | 9,90 | 8,90 |
| | | | | | | | | | | | 52,63 | 5,52 | 9,86 | 9,4 |
| 67 | $CH_3$- | H- | H- | H- | $CH_3$- | H- | $CH_3$- | H- | 59,3 | | 58,43 | 6,37 | 5,24 | 11,99 |
| | | | | | | | | | | | 58,56 | 6,52 | 5,22 | 12,23 |
| 68 | $CH_3$- | H- | H- | H- | $CH_3$- | H- | $CH_3$- | $CH_3$ CO - | 69 | | 58,25 | 6,15 | 4,53 | 10,36 |
| | | | | | | | | | | | 58,35 | 6,25 | 4,53 | 10,36 |
| 69 | $CH_3$- | H- | H- | H- | $CH_3$- | $CH_3$- | $(CH_3)_2CH$ - | $CH_3$ CO - | | 1,5235 | 61,54 | 7,12 | 3,99 | 9,12 |
| | | | | | | | | | | | 60,01 | 7,08 | 4,87 | 9,44 |
| 70 | $CH_3$- | H- | H- | H- | $CH_3$- | $CH_3$- | $(CH_3)_2CH - CH_2$- | $CH_3$ CO - | | 1,527 | 62,47 | 7,40 | 3,84 | 8,77 |
| | | | | | | | | | | | 63,05 | 7,75 | 3,96 | 8,57 |

Tableau II (suite)

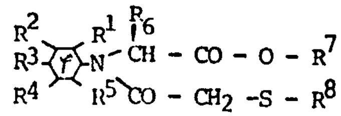

| Composé n° | $R^1$- | $R^2$- | $R^3$- | $R^4$- | $R^5$- | $R^6$- | $R^7$- | $R^8$- | Caractéristiques physiques: Point de fusion en °C | $n_D^{20}$ | analyse élémentaire 1°ligne : valeurs calculées 2°ligne : valeurs trouvées: C | H | N | S |
|---|---|---|---|---|---|---|---|---|---|---|---|---|---|---|
| 71 | $CH_3$- | H- | H- | H- | $CH_3$- | $CH_3$- | n - $C_6H_{13}$- | $CH_3$ CO - | | 1,521 | 64,12<br>64,79 | 7,89<br>7,81 | 3,56<br>3,56 | 8,14<br>8,06 |
| 72 | $CH_3$- | H- | H- | H- | $CH_3$- | $CH_3$- | n - $C_{12}H_{25}$- | $CH_3$ CO - | | 1,503 | | | | |
| 85 | $CH_3$- | Br | H- | H- | $CH_3$- | $CH_3$- | $CH_3$- | $CH_3CO$ - | | 1,562 | | | | |
| 86 | $CH_3$- | Br | H- | H- | $CH_3$- | $CH_3$- | $CH_3$- | H- | | 1,571 | | | | |
| 87 | $CH_3$- | Br | H- | H- | $CH_3$- | $CH_3$- | $CH_3$- | n-$C_9H_{19}$- CO - | | 1,530 | | | | |
| 88 | $CH_3$- | Cl | H- | H- | $CH_3$- | $CH_3$- | $CH_3$- | H- | | 1,557 | | | | |
| 89 | $CH_3$- | Cl | H- | H- | $CH_3$- | $CH_3$- | $CH_3$- | n-$C_9$ $H_{19}$-CO- | | 1,523 | | | | |
| 90 | $CH_3$- | Cl | H- | H- | $CH_3$- | H- | $CH_3$- | $CH_3$ CO - | | 1,555 | | | | |
| 91 | $CH_3$- | Cl- | H- | H- | $CH_3$- | H- | $CH_3$- | H- | 67,2 | | | | | |
| 92 | $CH_3$- | Cl- | H- | H- | $CH_3$- | H- | $CH_3$- | n-$C_9H_{19}$-CO- | | 1,528 | | | | |
| 93 | $CH_3$- | H- | H- | H- | $CH_3$- | H- | $CH_3$- | n-$C_9H_{19}$-CO- | | 1,518 | | | | |

Tableau III — Composés de formule

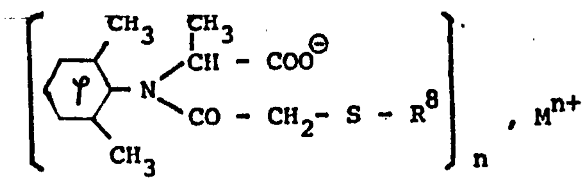

| | $R^8$ | n | $M^{n+}$ | Point de fusion en° C | Caractéristiques physiques | | | | | | | | | |
|---|---|---|---|---|---|---|---|---|---|---|---|---|---|---|
| | | | | | Analyse élémentaire | | | | | | | | | |
| | | | | | valeurs calculées en % | | | | | valeurs trouvées en % | | | | |
| | | | | | C | H | N | S | cation | C | H | N | S | cation |
| 73 | H- | 1 | $H^+$ | 152 | 58,43 | 6,37 | 5,24 | 11,99 | | 58,17 | 6,49 | 5,18 | 11,80 | |
| 74 | H- | 1 | $NH_4^+$ | | 54,97 | 7,04 | 9,86 | 11,27 | | 55,03 | 7,17 | 8,42 | 11,20 | |
| 75 | H- | 1 | $Na^+$ | 124,5 | 53,98 | 5,54 | 4,84 | 11,07 | | 52,47 | 5,96 | 4,78 | 11,1 | |
| 76 | H- | 2 | $Ca^{++}$ | 245 | 54,55 | 5,59 | 4,89 | 11,19 | 6,99 | 53,34 | 5,94 | 4,72 | 10,27 | 7,1 |
| 77 | H- | 2 | $Mg^{++}$ | | 56,12 | 5,70 | 5,03 | 11,51 | 4,32 | 53,09 | 5,93 | 4,82 | 12,35 | 4,3 |
| 78 | H- | 3 | $Al^{+++}$ | 201 | 56,73 | 5,82 | 5,11 | 11,68 | 3,28 | 53,51 | 6,17 | 4,79 | 11,71 | 3,23 |
| 79 | H- | 2 | $Cu^{++}$ | 120 | 52,39 | 5,37 | 4,70 | 10,75 | 10,66 | 51,11 | 5,51 | 4,48 | 10,47 | 11,2 |
| 80 | $CH_3CO$ - | 1 | $H^+$ | 140 | 58,25 | 6,15 | 4,53 | 10,36 | | 57,96 | 6,18 | 4,54 | 10,30 | |
| 81 | $CH_3CO$ - | 1 | $Na^+$ | 69 | 54,38 | 5,44 | 4,23 | 9,67 | 6,95 | 52,16 | 6,23 | 4,16 | 9,92 | 6,6 |
| 82 | $CH_3CO$ - | 2 | $Mg^{++}$ | 127 | 56,25 | 5,62 | 4,37 | 10 | 3,75 | 55,91 | 6,33 | 4,37 | 11,04 | 3,15 |
| 83 | $CH_3CO$ - | 3 | $Al^{+++}$ | 131 | 56,78 | 5,68 | 4,42 | 10,09 | 2,84 | 54,22 | 6,04 | 4,23 | 10,09 | 2,84 |
| 84 | $CH_3CO$ - | 2 | $Cu^{++}$ | 125 | 52,98 | 5,30 | 4,12 | 9,42 | 9,34 | 51,99 | 5,50 | 4,09 | 9,31 | 9,3 |

Exemple 11

Test in vivo sur Plasmopara viticola sur plants de vigne (traitement préventif)

Des plants de vigne (cépage GAMAY), cultivés en pots sont traités sur les deux faces de leurs feuilles par pulvérisation d'une solution aqueuse ou émulsion aqueuse contenant 1 mg/l de matière active à tester ; la solution ou émulsion pulvérisée est constituée de :

— 40 mg de matière active à tester
— 40 $cm^3$ d'eau
— 0,2 $cm^3$ de Tween 80 (agent tensioactif constitué d'un oléate de dérivé polyoxyéthyléné du sorbitol).

Cette solution ainsi constituée est diluée à l'aide d'eau pour obtenir des solutions à pulvériser de concentrations en matière active à tester inférieures à 1 mg/l.

Au bout de 48 heures, la contamination est effectuée par pulvérisation, sur la face inférieure des feuilles d'une suspension aqueuse de 80 000 unités/$cm^3$ environ de spores du champignon. Ensuite, les pots sont placés pendant 48 heures en cellule d'incubation à 100 % d'humidité relative et à 20 °C.

On effectue le contrôle des plants 9 jours après l'infestation.

Selon ce test tous les composés suivants ont assuré une protection totale lorsqu'ils ont été appliqués à la concentration de 0,11 g/l : 1 à 22, 24, 25, 29, 30, 33 à 36, 38 à 43, 45, 47 à 53, 55, 65 à 68 ; tous les composés suivants ont assuré une protection totale lorsqu'ils ont été appliqués à la concentration de 0,33 g/l : 44, 46, 56, 59, 62, 76, 81, 82, 84 ; tous les composés suivants ont assuré une protection totale lorsqu'ils ont été appliqués à la concentration de 1 g/l : 23, 26 à 28, 32, 37, 54, 57, 60, 69 à 72, 78, 79, 83 ; à cette même concentration de 1 g/l on a obtenu une bonne protection avec les composés 58, 61 et 74, une assez bonne protection avec le composé 73 et une protection moyenne avec les composés 63, 75, 77 et 80.

Parmi tous ces composés, certains (notamment le composé n° 1) présentent encore une assez bonne activité à des concentrations très inférieures, même à des concentrations de quelques mg/l.

Exemple 12

Test in vivo sur Erysiphe graminis sur orge (oïdium de l'orge)

De l'orge, en godets, semée dans de la terre franche, est traitée au stade 10 cm de hauteur par pulvérisation d'une solution aqueuse ou émulsion aqueuse de même composition que celle décrite à l'exemple 11 et de concentration égale à 1 g/l. L'essai est répété deux fois. Au bout de 48 heures, on saupoudre les plants d'orge avec des spores d'Erysiphe graminis, le saupoudrage étant effectué à l'aide de plants malades.

La lecture se fait 8 jours après la contamination.

Dans ces conditions, on observe une protection totale des plants d'orge avec le composé n° 47, une bonne protection avec le composé n° 6, et une assez bonne protection avec le composé n° 61.

Exemple 13

Test in vivo sur « Puccinia Striiformis » responsable de la rouille du blé

Du blé, en godets, semé dans de la terre franche, est traité au stade 10 cm de hauteur par pulvérisation d'une solution aqueuse ou émulsion aqueuse de même composition que celle décrite à l'exemple 11 et de concentration égale à 1 g/l. L'essai est répété 2 fois.

Au bout de 48 heures, une suspension aqueuse de spores (50 000 sp/$cm^3$) est pulvérisée sur le blé ; cette suspension a été obtenue à partir de plants contaminés. On place ensuite le blé pendant 48 heures en cellule climatique réglée de la façon suivante : éclairement : 16 h/jour ; température : 20 °C le jour et 15 °C la nuit ; humidité relative : 100 %.

Au bout de ces 2 jours, l'humidité relative est ramenée à 60 %. Le contrôle de l'état des plants se fait le 15$^e$ jour après la contamination par comparaison avec le témoin non traité.

Dans ces conditions on observe une protection totale du blé avec les composés n° 26 et 48 et une bonne protection avec les composés n° 15, 17, 25, 28, 30, 43, 52, 57, 60, 61, 71, 77.

Exemple 14

Test in vivo sur « Phytophthora Infestans » responsable du mildiou de la tomate

Des plants de tomates (variété Marmande) cultivés en serre et agés de 60 à 75 jours sont traités par pulvérisation avec des solutions aqueuses ou émulsions aqueuses préparées comme indiqué à l'exemple 11 et contenant diverses concentrations de matière active à tester.

Au bout de 48 heures, les plants traités sont contaminés avec une suspension aqueuse de spores

(zoosporanges) obtenue à partir d'une culture de « Phytophthora Infestans » cultivée pendant 20 jours sur un milieu à base de farine de pois chiches.

Les plants de tomate sont placés pendant 48 heures dans une enceinte à une température de 16 à 18 °C et munie d'une atmosphère ayant une humidité relative de 100 % puis l'humidité relative est ensuite ramenée à 80 %.

On observe les résultats 8 jours après contamination dans une cellule munie d'une atmosphère ayant une humidité relative de 80 %. Les résultats s'apprécient par évaluation de la surface de feuilles envahie par le champignon et s'expriment par le « pourcentage de protection » c'est-à-dire 100 (1-S/Stm), S étant la surface envahie par le champignon sur le plant considéré et Stm étant la surface envahie par le champignon sur le plant témoin non traité. On indique ci-après les résultats, comme dans les exemples précédents, sous forme de : protection totale, bonne, assez bonne ou moyenne.

Dans ces conditions et avec une solution aqueuse de concentration 1 g/l en matière active à tester, on a observé une protection totale avec les composés n° 2, 3, 12, 14, 15, 19, 22, 23, 31, 50, 52 et une bonne protection avec les composés n° 17 et 45.

A la concentration de 0,11 g/l on a observé une protection totale avec les composés n° 16 et 66, une bonne protection avec les composés n° 24 et 38.

Ces expérimentations illustrent clairement les remarquables propriétés fongicides des composés selon l'invention, spécialement sur les champignons de type Phycomycètes (mildiou) ainsi que leur absence de phytotoxicité ; leur activité élevée à des doses mêmes faibles est tout à fait remarquable. Ces composés peuvent donc être utilisés pour la lutte, tant préventive que curative contre les maladies fongiques (spécialement les mildious) des végétaux ou des plantes en général et, en particulier de la vigne, du tabac, du houblon, de la tomate, de la pomme de terre, du tournesol et des cultures maraîchères en général.

Par ailleurs, les composés de l'invention peuvent encore être appliqués contre les maladies fongiques d'autres végétaux notamment les céréales, et plus spécialement le blé et l'orge.

Pour leur emploi pratique les composés selon l'invention sont rarement utilisés seuls. Le plus souvent ils font partie de compositions. Ces compositions, utilisables pour la protection des végétaux contre les maladies fongiques, contiennent comme matière active un composé selon l'invention tel que décrit précédemment en association avec les supports solides ou liquides, acceptables en agriculture et les agents tensio-actifs également acceptables en agriculture. En particulier sont utilisables les supports inertes et usuels et les agents tensio-actifs usuels.

Ces compositions peuvent contenir aussi toute sorte d'autres ingrédients tels que, par exemple, des colloïdes protecteurs, des adhésifs, des épaississants, des agents thixotropes, des agents de pénétration, des stabilisants, des séquestrants, etc. ainsi que d'autres matières actives connues à propriétés pesticides (notamment insecticides ou fongicides) ou à propriétés favorisant la croissance des plantes (notamment des engrais) ou à propriétés régulatrices de la croissance des plantes. Plus généralement les composés selon l'invention peuvent être associés à tous les additifs solides ou liquides correspondant aux techniques habituelles de la mise en formulation.

Les doses d'emploi des composés selon l'invention peuvent varier dans de larges limites, notamment selon la virulence des champignons et les conditions climatiques.

D'une manière générale des compositions contenant 0,5 à 5 000 ppm de substance active conviennent bien ; ces valeurs sont indiquées pour les compositions prêtes à l'application. Ppm signifie « partie par million ». La zone de 0,5 à 5 000 ppm correspond à une zone de $5 \cdot 10^{-5}$ à 0,5 % (pourcentage pondéraux).

En ce qui concerne les compositions adaptées au stockage et au transport, elles contiennent plus avantageusement de 10 à 95 % (en poids) de substance active.

Ainsi donc, les compositions à usage agricole selon l'invention peuvent contenir les matières actives selon l'invention dans de très larges limites, allant de $5 \cdot 10^{-5}$ % à 95 % (en poids).

Selon ce qui a déjà été dit les composés selon l'invention sont généralement associés à des supports et éventuellement des agents tensioactifs.

Par le terme « support », dans le présent exposé, on désigne une matière organique ou minérale, naturelle ou synthétique, avec laquelle la matière active est associée pour faciliter son application sur la plante, sur des graines ou sur le sol. Ce support est donc généralement inerte et il doit être acceptable en agriculture, notamment sur la plante traitée. Le support peut être solide (argiles, silicates naturels ou synthétiques, silice, résines, cires, engrais solides, etc...) ou liquide (eau, alcools, cétones, fractions de pétrole, hydrocarbures aromatiques ou paraffiniques, hydrocarbures chlorés, gaz liquéfiés, etc...).

L'agent tensioactif peut être un agent émulsionnant, dispersant ou mouillant, de type ionique ou non ionique. On peut citer par exemple des sels d'acides polyacryliques, des sels d'acides lignosulfoniques, des sels d'acides phénolsulfoniques ou naphtalènesulfoniques, des polycondensats d'oxyde d'éthylène sur des alcools gras ou sur des acides gras ou sur des amines grasses, des phénols substitués (notamment des alkylphénols ou des arylphénols), des sels d'esters d'acides sulfosucciniques, des dérivés de la taurine (notamment des alkyltaurates), des esters phosphoriques d'alcools ou de phénols polyoxyéthylés. La présence d'au moins un agent tensioactif est généralement indispensable lorsque la matière active et/ou le support inerte ne sont pas solubles dans l'eau et que l'agent vecteur de l'application est l'eau.

Pour leur application, les composés de formule (I) se trouvent donc généralement sous forme de compositions ; ces compositions selon l'invention sont elles-mêmes sous des formes assez diverses, solides ou liquides.

Comme formes de compositions solides, on peut citer les poudres pour poudrage ou dispersion (à teneur en composé de formule (I) pouvant aller jusqu'à 100 %) et les granulés, notamment ceux obtenus par extrusion, par compactage, par imprégnation d'un support granulé, par granulation à partir d'une poudre (la teneur en composé de formule (I) dans ces granulés étant entre 1 et 80 % pour ces derniers cas).

Comme formes de compositions liquides ou destinées à constituer des compositions liquides lors de l'application, on peut citer les solutions, en particulier les concentrés solubles dans l'eau, les concentrés émulsionnables, les émulsions, les suspensions concentrées, les aérosols, les poudres mouillables (ou poudres à pulvériser), les pâtes.

Les concentrés émulsionnables ou solubles comprennent le plus souvent 10 à 80 % de matière active, les émulsions ou solutions prêtes à l'application contenant, quant à elles, 0,01 à 20 % de matière active. En plus du solvant, les concentrés émulsionnables peuvent contenir, quand c'est nécessaire, 2 à 20 % d'additifs appropriés, comme des stabilisants, des agents tensioactifs, des agents de pénétration, des inhibiteurs de corrosion, des colorants, des adhésifs. A titre d'exemple, voici la composition de quelques concentrés :

| | |
|---|---|
| — matière active | 400 g/l |
| — dodécylbenzène sulfonate alcalin | 24 g/l |
| — nonylphénol oxyéthylé à 10 molécules d'oxyde d'éthylène | 16 g/l |
| — cyclohexanone | 200 g/l |
| — solvant aromatique | q.s.p. 1 litre |

Selon une autre formule de concentré émulsionnable, on utilise :

| | |
|---|---|
| — matière active | 250 g |
| — huile végétale époxydée | 25 g |
| — mélange de sulfonate d'alcoylaryle et d'éther de polyglycol et d'acools gras | 100 g |
| — diméthylformamide | 50 g |
| — xylène | 575 g |

A partir de ces concentrés, on peut obtenir par dilution avec de l'eau des émulsions de toute concentration désirée, qui conviennent particulièrement à l'application sur les feuilles.

Les suspensions concentrées, également applicables en pulvérisation, sont préparées de manière à obtenir un produit fluide stable ne se déposant pas et elles contiennent habituellement de 10 à 75 % de matière active, de 0,5 à 15 % d'agents tensioactifs, de 0,1 à 10 % d'agents thixotropes, de 0 à 10 % d'additifs appropriés, comme des anti-mousses, des inhibiteurs de corrosion, des stabilisants, des agents de pénétration et des adhésifs et, comme support, de l'eau ou un liquide organique dans lequel la matière active est peu ou pas soluble : certaines matières solides organiques ou des sels minéraux peuvent être dissous dans le support pour aider à empêcher la sédimentation ou comme antigels pour l'eau.

Les poudres mouillables (ou poudres à pulvériser) sont habituellement préparées de manière qu'elles contiennent 20 à 95 % de matière active, et elles contiennent habituellement, en plus du support solide, de 0 à 5 % d'un agent mouillant, de 3 à 10 % d'un agent dispersant, et, quand c'est nécessaire, de 0 à 10 % d'un ou plusieurs stabilisants et/ou autres additifs, comme des agents de pénétration, des adhésifs, ou des agents antimottants, colorants, etc...

A titre d'exemple, voici diverses compositions de poudres mouillables :

| | |
|---|---|
| — matière active | 50 % |
| — lignosulfonate de calcium (défloculant) | 5 % |
| — isopropylnaphtalène sulfonate (agent mouillant anionique) | 1 % |
| — silice antimottante | 5 % |
| — kaolin (charge) | 39 % |

Une autre composition de poudre à pulvériser à 70 % utilise les constituants suivants :

| | |
|---|---|
| — matière active | 700 g |
| — dibutylnaphtylsulfonate de sodium | 50 g |
| — produit de condensation en proportions 3/2/1 d'acide naphtalène sulfonique, d'acide phénolsulfonique et de formaldéhyde | 30 g |
| — kaolin | 100 g |
| — craie de Champagne | 120 g |

Une autre composition de poudre à pulvériser à 40 % utilise les constituants suivants :

| | |
|---|---|
| — matière active | 400 g |
| — lignosulfonate de sodium | 50 g |
| — dibutylnaphtalène sulfonate de sodium | 10 g |
| — silice | 540 g |

Une autre composition de poudre à pulvériser à 25 % utilise les constituants suivants :

| | |
|---|---|
| — matière active | 250 g |
| — lignosulfonate de calcium | 45 g |
| — mélange équipondéral de craie de Champagne et d'hydroxyéthylcellulose | 19 g |
| — dibutylnaphtalène sulfonate de sodium | 15 g |
| — silice | 195 g |
| — craie de Champagne | 195 g |
| — kaolin | 281 g |

Une autre composition de poudre à pulvériser à 25 % utilise les constituants suivants :

| | |
|---|---|
| — matière active | 250 g |
| — isooctylphénoxy-polyoxyéthylène-éthanol | 25 g |
| — mélange équipondéral de craie de Champagne et d'hydroxyéthylcellulose | 17 g |
| — aluminosilicate de sodium | 543 g |
| — kieselguhr | 165 g |

Une autre composition de poudre à pulvériser à 10 % utilise les constituants suivants :

| | |
|---|---|
| — matière active | 100 g |
| — mélange de sels de sodium de sulfates d'acides gras saturés | 30 g |
| — produit de condensation d'acide naphtalène sulfonique et de formaldéhyde | 50 g |
| — kaolin | 820 g |

Pour obtenir ces poudres à pulvériser ou poudres mouillables, on mélange intimement les matières actives dans des mélangeurs appropriés avec les substances additionnelles et on broie avec des moulins ou autres broyeurs appropriés. On obtient par là des poudres à pulvériser dont la mouillabilité et la mise en suspension sont avantageuses ; on peut les mettre en suspension avec de l'eau à toute concentration désirée et cette suspension est utilisable très avantageusement en particulier pour l'application sur les feuilles des végétaux.

A la place des poudres mouillables, on peut réaliser des pâtes. Les conditions et modalités de réalisation et d'utilisation de ces pâtes sont semblables à celles des poudres mouillables ou poudres à pulvériser.

Comme cela a déjà été dit, les dispersions et émulsions aqueuses, par exemple des compositions obtenues en diluant à l'aide d'eau une poudre mouillable ou un concentré émulsionnable selon l'invention, sont comprises dans le cadre général de la présente invention. Les émulsions peuvent être du type eau-dans-l'huile ou huile-dans-l'eau et elles peuvent avoir une consistance épaisse comme celle d'une « mayonnaise ».

Les granulés destinés à être disposés sur le sol sont habituellement préparés de manière qu'ils aient des dimensions comprises entre 0,1 et 2 mm et ils peuvent être fabriqués par agglomération ou imprégnation. En général, les granulés contiennent 0,5 à 25 % de matière active et 0 à 10 % d'additifs comme des stabilisants, des agents de modification à libération lente, des liants et des solvants.

Selon un exemple de composition de granulé, on utilise les constituants suivants :

| | |
|---|---|
| — matière active | 50 g |
| — épichlorhydrine | 2,5 g |
| — éther de cétyle et de polyglycol | 2,5 g |
| — polyéthylène glycol | 35 g |
| — kaolin (granulométrie : 0,3 à 0,8 mm) | 910 g |

Dans ce cas particulier on mélange la matière active avec l'épichlorhydrine et on dissout avec 60 g d'acétone ; on ajoute alors le polyéthylène glycol et l'éther de cétyle et de polyglycol. On arrose le kaolin avec la solution obtenue et on évapore ensuire l'acétone sous vide. On utilise avantageusement un tel microgranulé pour lutter contre les champignons du sol.

Les composés de formule (I) peuvent encore être utilisés sous forme de poudres pour poudrage ; on peut aussi utiliser une composition comprenant 50 g de matière active et 950 g de talc ; on peut aussi utiliser une composition comprenant 20 g de matière active, 10 g de silice finement divisée et 970 g de talc ; on mélange et broie ces constituants et on applique le mélange par poudrage.

**Revendications**

1. Produits, utilisables notamment comme agent pesticide, caractérisés en ce qu'ils ont pour formule :

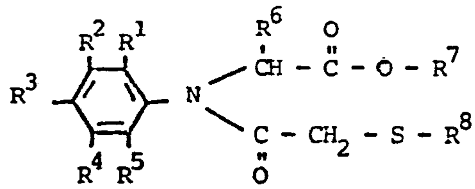

(I)

dans laquelle :

$R^1$ et $R^5$, identiques ou différents, représentent un radical alkyle ou alkoxyle ayant de 1 à 4 atomes de carbone ou un atome d'halogène ou l'atome d'hydrogène, un seul des deux radicaux $R^1$ ou $R^5$ pouvant avoir cette dernière signification.

$R^2$, $R^3$ et $R^4$, identiques ou différents, représentent l'atome d'hydrogène ou un atome d'halogène ou un radical alkyle ou alkoxyle ayant de 1 à 4 atomes de carbone,

$R^6$ représente l'atome d'hydrogène ou un raical méthyle,

$R^7$ représente l'atome d'hydrogène ou un radical alcoyle ayant de 1 à 12 atomes de carbone ou 1/n $M^{n+}$, M étant un cation de valence n, plus particulièrement un cation d'ammonium (éventuellement substitué) ou un cation métallique,

$R^8$ représente l'atome d'hydrogène ou un radical de formule $R^9$—CO— dans laquelle $R^9$ est un radical organique qui représente

— un radical alkyle ou alkényle, linéaire ou ramifié, ayant au plus 18 atomes de carbone et pouvant être substitué par des atomes d'halogènes ou des groupes hydroxy, mercaptan, cyano, oxo ou des radicaux alkyloxy, alkylthio, aryle, alkoxycarbonyle, acylamino, ces divers radicaux ayant au plus 6 atomes de carbone, ou,

— un radical cycloalkyle comportant 3 à 7 atomes de carbone dans le cycle et pouvant être substitué par des atomes d'halogène ou des groupements alkyle ayant au plus 4 atomes de carbone, ou,

— un radical phényle ou naphtyle, éventuellement substitué par des atomes d'halogène ou des groupements alkyle, acyloxyle, acyle, alkoxyle, ces divers groupements ayant au plus 6 atomes de carbone, ou,

— un radical hétérocyclique comportant, dans le cycle, 5 ou 6 atomes dont 1 à 3 sont des hétéroatomes d'oxygène, d'azote ou de soufre, ce radical hétérocyclique pouvant être substitué par des atomes de chlore ou des groupements méthyle ou éthyle, et la valence libre de ce radical $R^9$ (c'est-à-dire la valence rattachée au groupe carbonyle dans le radical $R^9$—CO—) est portée par un atome de carbone, ou,

— un radical comprenant un groupe hétérocyclique ayant la même signification que celle indiquée dans le paragraphe précédent, ce groupe hétérocyclique étant relié au groupe carbonyle (de $R^9$—CO—) par l'intermédiaire d'un radical divalent méthylène ou éthylène.

2. Produits selon la revendication 1 caractérisés en ce que $R^7$ représente un groupe alcoyle de 1 à 12 atomes de carbone et que les atomes d'halogène sont le chlore ou le brome.

3. Produits selon l'une des revendications 1 ou 2 caractérisés en ce que :

$R^2$ représente un atome d'hydrogène, de chlore ou de brome

$R^3$ et $R^4$ représentent l'atome d'hydrogène

$R^1$ et $R^5$ représentent un groupe alcoyle ayant de 1 à 4 atomes de carbone.

4. Produits selon l'une des revendications 1 à 3 caractérisés en ce que $R^9$ représente :

— un radical alkyle ou alkényle éventuellement substitué par des atomes de chlore ou des groupes alkoxyle ayant de 1 à 4 atomes de carbone ou des groupes cyano, oxo, acétamido, phényle, chlorophényle, alkylthio, alkoxycarbonyle

— un radical cyclopropyle, cyclopentyle ou cyclohexyle

— un radical phényle éventuellement substitué par un atome de chlore ou de brome

— un radical furyle, thiényle, trichlorothiényle, pyridyle.

5. Produits selon l'une des revendications 1 à 4 caractérisés en ce que $R^1$, $R^5$ et $R^7$ sont le radical méthyle et $R^9$ est un radical alkyle ayant de 1 à 9 atomes de carbone.

6. Procédé de préparation de composés de formule

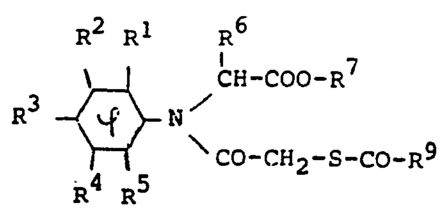

(III)

caractérisé en ce que l'on fait réagir un dérivé de N-phénylaminoacide de formule

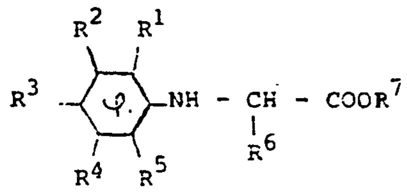

(IV)

avec un chlorure d'acide de formule $R^9$—CO—S—$CH_2$—CO—Cl (V), les symboles $R^1$ à $R^6$ et $R^9$ ayant les significations données dans l'une des revendications 1 à 5 et le symbole $R^7$ représentant l'atome d'hydrogène ou un radical alkyle.

7. Procédé selon la revendication 6 caractérisé en ce que la réaction est effectuée dans un solvant inerte, à température comprise entre 40 °C et la température de dégradation des réactifs et/ou produits de réaction.

8. Procédé de préparation de produits de formule

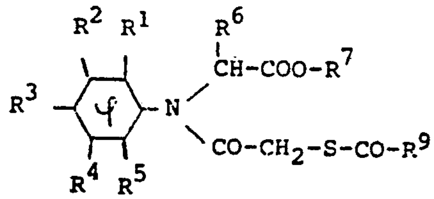

(III)

caractérisé en ce que l'on fait réagir un dérivé de N-phénylaminoacide de formule

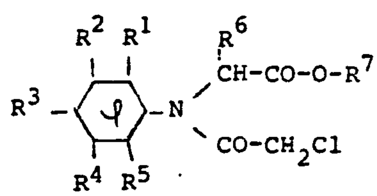

(IV)

avec un thiocarboxylate dérivé d'un acide thiocarboxylique de formule $R^9$—CO—SH dans lesquelles $R^1$ à $R^7$ et $R^9$ ont les significations données dans l'une des revendications 1 à 5.

9. Procédé selon la revendication 8, caractérisé en ce que le thiocarboxylate est un thiocarboxylate alcalin ou d'ammonium ou alcalino-terreux et que la réaction est effectuée dans un solvant inerte à température comprise entre 40 °C et la température de dégradation des réactifs et/ou produits de réaction.

10. Procédé de préparation des composés produits selon l'une des revendications 1 à 5 et ayant pour formule :

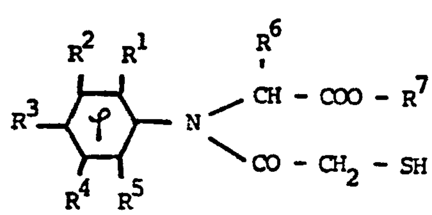

(VIII)

caractérisé en ce qu'on fait réagir un composé de formule :

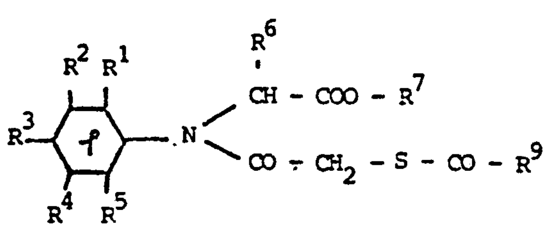

avec un alcool

11. Procédé selon la revendication 10 caractérisé en ce que l'alcool a pour formule $R^7OH$, $R^7$ étant un radical alkyle.

12. Procédé selon l'une des revendications 10 ou 11 caractérisé en ce que l'alcool est du méthanol.

13. Procédé selon l'une des revendications 10 à 12 caractérisé en ce que la réaction est effectuée en présence d'un alcoolate alcalin, de préférence le méthanolate de sodium ou potassium.

14. Procédé selon l'une des revendications 10 à 12 caractérisé en ce que la réaction est effectuée en présence d'un acide fort, minéral ou organique.

15. Procédé de préparation de produits de formule

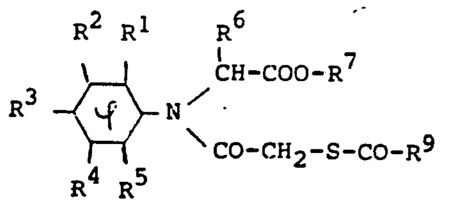

(III)

caractérisé en ce que l'on fait réagir un mercaptan de formule

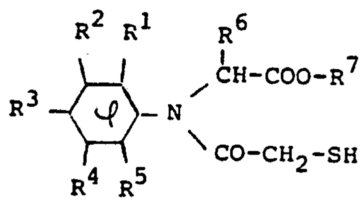

avec un chlorure ou anhydride d'acide de formule $R^9$—COOH, les symboles de ces diverses formules étant tels que $R^1$ à $R^7$ et $R^9$ ont les significations données dans l'une des revendications 1 à 5.

16. Procédé selon la revendication 15 caractérisé en ce que l'on utilise un chlorure d'acide de formule $R^9$ COCl et en ce que la réaction est effectuée dans un solvant inerte en présence d'un accepteur d'acide.

17. Procédé selon la revendication 15 caractérisé en ce que l'on utilise un anhydride d'acide de formule $(R^9CO)_2O$ préparé in situ à l'aide de l'acide $R^9COOH$ et d'un agent de déshydratation.

18. Compositions, utilisables notamment pour la protection des végétaux contre les maladies fongiques, caractérisées en ce qu'elles contiennent, comme matière active, un composé selon l'une des revendications 1 à 5 en association avec les supports inertes et usuels et éventuellement les agents tensio-actifs usuels.

19. Compositions selon la revendication 18, caractérisées en ce qu'elles contiennent $5 \cdot 10^{-5}$ à 95 % de matière active.

20. Procédé de lutte contre les maladies fongiques des plantes caractérisé en ce que l'on utilise une composition selon l'une des revendications 18 ou 19.

**Claims**

1. A product, which can be used in particular as a pesticide, of the formula :

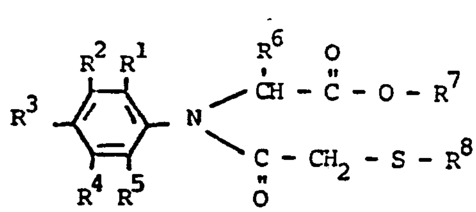

(I)

in which

$R^1$ and $R^5$, which are identical or different, represent an alkyl or alkoxy radical having from 1 to 4 carbon atoms or a halogen atom or the hydrogen atom, it being possible for only one of two radicals $R^1$ or $R^5$ to have this last meaning,

$R^2$, $R^3$ and $R^4$, which are identical or different, represent the hydrogen atom or a halogen atom or an lakyl or alkoxy radical having from 1 to 4 carbon atoms,

$R^6$ represents the hydrogen atom or a methyl radical,

$R^7$ represents the hydrogen atom or an alkyl radical having from 1 to 12 carbon atoms or $1/n\ M^{n+}$, M being a cation of valency n and more particularly an ammonium cation (which is optionally substituted) or a metal cation, and

$R^8$ represents the hydrogen atom or a radical of the formula $R^9$—CO—, in which $R^9$ is an organic radical which represents

— a linear or branched alkyl or alkenyl radical which has at most 18 carbon atoms and can be substituted by halogen atoms or hydroxyl, mercaptan, cyano or oxo groups or alkoxy, alkylthio, aryl, alkoxycarbonyl or acylamino radicals, these various radicals having at most 6 carbon atoms,

— a cycloalkyl radical which contains 3 to 7 carbon atoms in the ring and can be substituted by halogen atoms or alkyl groups having at most 4 carbon atoms,

— a phenyl or naphthyl radical which is optionally substituted by halogen atoms or alkyl, acyloxy, acyl or alkoxy groups, these various groups having at most 6 carbon atoms,

— a heterocyclic radical containing, in the ring, 5 or 6 atoms of which 1 to 3 are oxygen, nitrogen or sulphur hetero-atoms, it being possible for this heterocyclic radical to be substituted by chlorine atoms or methyl or ethyl groups, and the free valency of this radical $R^9$ (i.e. the valency attached to the carbonyl group in the radical $R^9$—CO—) being by a carbon atom, or

— a radical comprising a heterocyclic group having the same meaning as that indicated in the preceding definition, this heterocyclic group being joined to the carbonyl group (of $R^9$—CO—) via a divalent methyle or ethylene radical.

2. A product according to claim 1, in which $R^7$ represents an alkyl group having 1 to 12 carbon atoms and the halogen atoms are chlorine or bromine.

3. A product according to one of claims 1 or 2, in which

$R^2$ represents a hydrogen, chlorine or bromine atom,

$R^3$ and $R^4$ represent the hydrogen atom and

$R^1$ and $R^5$ represent an alkyl group having from 1 to 4 carbon atoms.

4. A product according to one of claims 1 to 3 in which $R^9$ represents

— an alkyl or alkenyl radical which is optionally substituted by chlorine substituted by chlorine atoms or alkoxy groups having from 1 to 4 carbon atoms or cyano, oxo, acetamido, phenyl, chlorophenyl, alkylthio or alkoxycarbonyl groups,

— a cyclopropyl, cyclopentyl or cyclohexyl radical,

— a phenyl radical which is optionally substituted by a chlorine or bromine atom, or

— a furyl, thienyl, trichlorothienyl or pyridyl radical.

5. A product according to one of claims 1 to 4, in which $R^1$, $R^5$ and $R^7$ are the methyl radical and $R^9$ is an alkyl radical having from 1 to 9 carbon atoms.

6. A process for the preparation of a compound of the formula

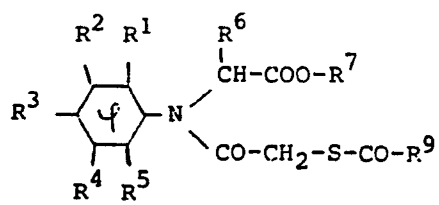

(III)

which comprises reacting a N-phenyl-aminoacid derivative of the formula

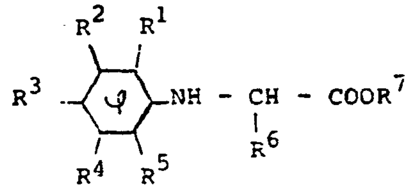

(IV)

with an acid chloride of the formula $R^9$—CO—S—$CH_2$—CO—Cl (V), the symbols $R^1$ to $R^6$ and $R^9$ having the meanings given in one of claims 1 to 5 and the symbol $R^7$ representing the hydrogen atom or an alkyl radical.

7. A process according to claim 6, wherein the reaction is carried out in an inert solvent at a temperature between 40 °C and the degradation temperature of the reactants and/or reaction products.

8. A process for the preparation of a product of the formula

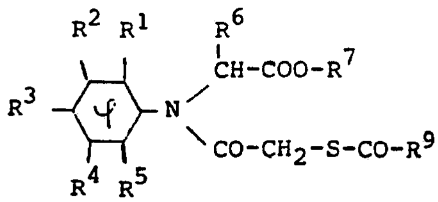

(III)

which comprises reacting a N-phenyl-aminoacid derivative of the formula

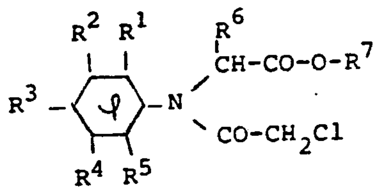

(IV)

with a thiocarboxylate derived from a thiocarboxylic acid of the formula $R^9$—CO—SH, in which formulae $R^1$ to $R^7$ and $R^9$ have the meanings given in one of claims 1 to 5.

9. A process according to claim 8, wherein the thiocarboxylate is an alkali metal thiocarboxylate or ammonium thiocarboxylate or alkaline earth metal thiocarboxylate and the reaction is carried out in an inert solvent at a temperature between 40 °C and the degradation temperature of the reactants and/or reaction products.

10. A process for the preparation of a product according to one of claims 1 to 5, of the formula :

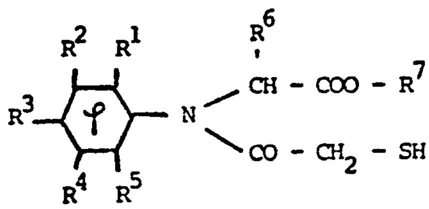

(VIII)

which comprises reacting a compound of the formula :

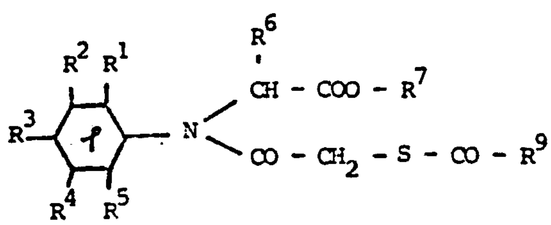

with an alcohol.

11. A process according to claim 10, wherein the alcohol has the formula $R^7OH$, $R^7$ being an alkyl radical.

12. A process according to one of claims 10 or 11, wherein the alcohol is methanol.

13. A process according to one of claims 10 to 12, wherein the reaction is carried out in the presence of an alkali metal alcoholate, preferably sodium methanolate or potassium methanolate.

14. A process according to one of claims 10 to 12, wherein the reaction is carried out in the presence of a strong mineral or organic acid.

15. A process for the preparation of a product of the formula

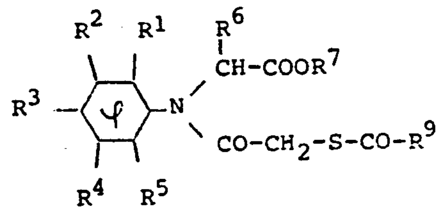

(III)

which comprises reacting a mercaptan of the formula

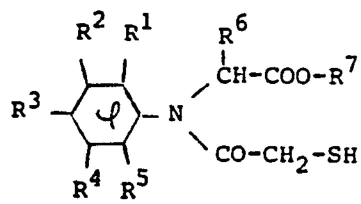

with the chloride or anhydride of an acid of the formula $R^9$—COOH, the symbols in these various formulae being such that $R^1$ to $R^7$ and $R^9$ have the meanings given in one of claims 1 to 5.

16. A process according to claim 15, wherein an acid chloride of the formula $R^9COCl$ is used and the reaction is carried out in an inert solvent in the presence of an acid acceptor.

17. A process according to claim 15, wherein an acid anhydride of the formula $(R^9CO)_2O$, prepared in situ using the acid $R^9COOH$ and a dehydrating agent, is used.

18. A composition which can be used in particular for protecting plants against fungal diseases and which contains, as the active ingredient, a compound according to one of claims 1 to 5, in association with the customary inert carriers and, if appropriate, the customary surface-active agents.

19. A composition according to claim 18, which contains $5 \cdot 10^{-5}$ to 95 % of active ingredient.

20. A process for combating fungal diseases in plants, wherein a composition according to one of claims 18 or 19 is used.

**Ansprüche**

1. Insbesondere als Schädlingsbekämpfungsmittel verwendbare Verbindungen der Formel

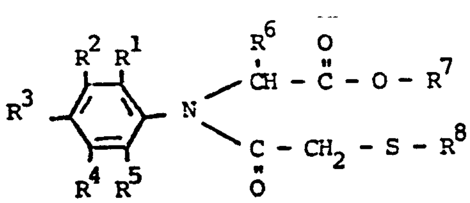

(I)

worin die einzelnen Symbole die folgenden Bedeutungen besitzen :

$R^1$ und $R^5$, welche identisch oder voneinander verschieden sind, bedeuten einen Alkyl- oder Alkoxyrest mit 1 bis 4 Kohlenstoffatomen oder ein Halogen atom oder das Wasserstoffatom, wobei nur einer dieser beiden Reste $R^1$ oder $R^5$ die letztgenannte Bedeutung haben kann ;

$R^2$, $R^3$ und $R^4$, welche identisch oder voneinander verschieden sind, bedeuten ein Wasserstoff- oder Halogenatom oder einen Alkyl- oder Alkoxyrest mit 1 bis 4 Kohlenstoffatomen ;

$R^6$ bedeutet ein Wasserstoffatom oder einen Methylrest ;

$R^7$ bedeutet ein Wasserstoffatom oder einen Alkylrest mit 1 bis 12 Kohlenstoffatomen oder $1/nM^{n+}$, wobei M ein Kation der Valenz n ist, insbesondere ein Ammoniumkation (gegebenenfalls substituiert) oder ein Metallkation ;

$R^8$ bedeutet das Wasserstoffatom oder einen Rest der Formel $R^9$—CO—, worin $R^9$ ein organischer Rest ist, der bedeutet

— einen geraden oder verzweigten Alkyl- oder Alkenylrest mit höchstens 18 Kohlenstoffatomen, der durch Halogenatome oder Hydroxy-, Mercaptan-, Cyano- oder Oxogruppen oder Alkoxy-, Alkylthio-, Aryl-, Alkoxycarbonyl- oder Acylaminoreste substituiert sein kann, wobei diese verschiedenen Reste höchstens 6 Kohlenstoffatome haben oder

— einen Cycloalkylrest mit 3 bis 7 Kohlenstoffatomen im Ring, der durch Halogenatome oder Alkylgruppen mit höchstens 4 Kohlenstoffatomen substituiert sein kann oder

— einen Phenyl- oder Naphthylrest, der gegebenenfalls durch Halogenatome oder Alkyl-, Acyloxy-,

Acyl- oder Alkoxylreste substituiert ist, wobei diese verschiedenen Gruppen höchstens 6 Kohlenstoffatome haben oder

— einen heterocyclischen Rest mit 5 oder 6 Heteroatomen im Ring, wovon 1 bis 3 Heteroatome vom Sauerstoff, Stickstoff oder Schwefel sind, wobei dieser heterocyclische Rest durch Chloratome oder Methyl- oder Äthylgruppen substituiert sein kann, und die freie Valenz dieses Restes $R^9$ (d.h. die Valenz, welche an die Carbonylgruppe in dem Rest $R^9$—CO— geknüpft ist) durch ein Kohlenstoffatom getragen ist oder

— einen Rest umfassend eine heterocliscche Gruppe mit derselben Bedeutung wie derjenigen in dem vorhergehenden Absatz, wobei diese heterocyclische Gruppe mit der Carbonylgruppe von $R^9$—CO— über einen zweiwertigen Methylen- oder Äthylenrest als Zwischenglied verbunden ist.

2. Verbindungen gemäß Anspruch 1, dadurch gekennzeichnet, daß $R^7$ eine Alkylgruppe mit 1 bis 12 Kohlenstoffatomen bedeutet und die Halogenatome Chlor oder Brom sind.

3. Verbindungen gemäß den Ansprüchen 1 oder 2, dadurch gekennzeichnet, daß

$R^2$ ein Wasserstoff-, Chlor- oder Bromatom,

$R^3$ und $R^4$ ein Wasserstoffatom,

$R^1$ und $R^5$ eine Alkylgruppe mit 1 bis 4 Kohlenstoffatomen bedeuten.

4. Verbindungen gemäß einem der Ansprüche 1 bis 3, dadurch gekennzeichnet, daß $R^9$ bedeutet :

— einen Alkyl- oder Alkenylrest, der gegebenenfalls durch Chloratome oder Alkoxygruppen mit 1 bis 4 Kohlenstoffatomen oder Cyano-, Oxo-, Acetamido-, Phenyl-, Chlorphenyl-, Alkylthio- oder Alkoxycarbonylgruppen substituiert ist,

— einen Cyclopropyl-, Cyclopentyl- oder Cyclohexylrest,

— einen Phenylrest, der gegebenenfalls durch ein Chlor- oder Bromatom substituiert ist,

— einen Furyl-, Thienyl-, Trichlorthienyl- oder Pyridylrest.

5. Verbindungen gemäß einem der Ansprüche 1 bis 4, dadurch gekennzeichnet, daß $R^1$, $R^5$ und $R^7$ den Methylrest darstellen und $R^9$ ein Alkylrest mit 1 bis 9 Kohlenstoffatomen ist.

6. Verfahren zur Herstellung von Verbindungen der Formel (III)

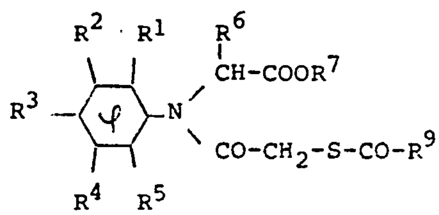

(III)

dadurch gekennzeichnet, daß ein N-Phenylaminosäurederivat der Formel

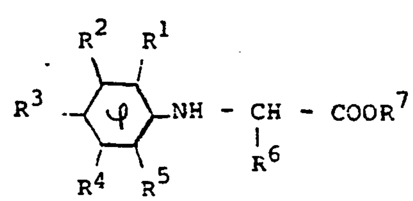

(IV)

mit einem Säurechlorid der Formel $R^9$—CO—S—$CH_2$—CO—Cl (V) umsetzt, wobei die Symbole $R^1$ bis $R^6$ und $R^9$ die in einem der Ansprüche 1 bis 5 angegebenen Bedeutungen besitzen und das Symbol $R^7$ ein Wasserstoffatom oder einen Alkylrest bedeutet.

7. Verfahren gemäß Anspruch 6, dadurch gekennzeichnet, daß die Umsetzung in einem inerten Lösungsmittel bei einer Temperatur zwischen 40° und der Zersetzungstemperatur der Reaktionsteilnehmer und/oder der Reaktionsprodukte durchgeführt wird.

8. Verfahren zur Herstellung der Verbindungen der Formel (III)

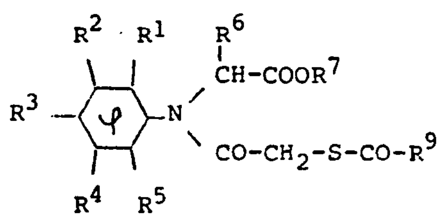

(III)

dadurch gekennzeichnet, daß man ein N-Phenylaminosäurederivat der Formel (IV)

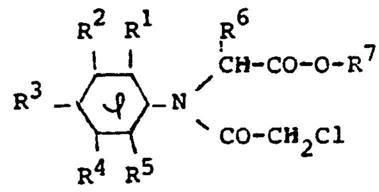

(IV)

mit einem Thiocarboxylat, das sich von einer Thiocarboxylsäure bzw. Thiocarbonsäure der Formel $R^9$—CO—SH ableitet, worin $R^1$ bis $R^7$ und $R^9$ die in einem Ansprüche 1 bis 5 angegebenen Bedeutungen besitzt.

9. Verfahren nach Anspruch 8, dadurch gekennzeichnet, daß das Thiocarboxylat ein Alkalithiocarboxylat oder Ammoniumthiocarboxylat oder Erdalkalithiocarboxylat ist und daß die Reaktion in einem inerten Lösungsmittel bei einer Temperatur zwischen 40 °C und der Zersetzungstemperatur der Reaktionsteilnehmer und/oder der Reaktionsprodukte durchgeführt wird.

10. Verfahren zur Herstellung der Verbindungen gemäß einem der Ansprüche 1 bis 5 der Formel (VIII)

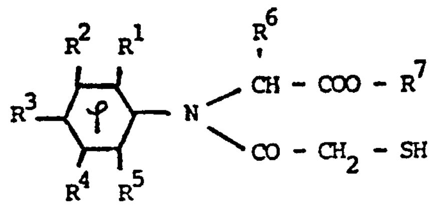

(VIII)

dadurch gekennzeichnet, daß man eine Verbindung der Formel

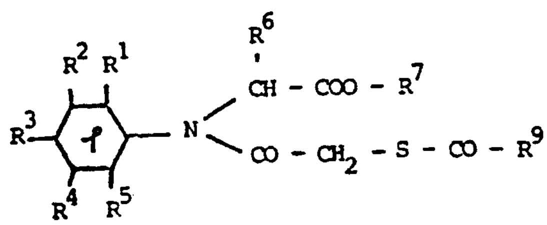

mit einem Alkohol umsetzt.

11. Verfahren gemäß Anspruch 10, dadurch gekennzeichnet, daß der Alkohol die Formel $R^7$—OH, hat wobei $R^7$ einen Alkylrest bedeutet.

12. Verfahren gemäß den Ansprüchen 10 oder 11, dadurch gekennzeichnet, daß man als Alkohol Methanol verwendet.

13. Verfahren nach einem der Ansprüche 10 bis 12, dadurch gekennzeichnet, daß die Reaktion in Anwesenheit eines Alkalialkoholats, vorzugsweise Natrium- oder Kaliummethanolat, durchgeführt wird.

14. Verfahren nach einem der Ansprüche 10 bis 12, dadurch gekennzeichnet, daß die Reaktion in Gegenwart einer starken Mineral- oder organischen Säure durchgeführt wird.

15. Verfahren zur Herstellung der Verbindungen der Formel (III)

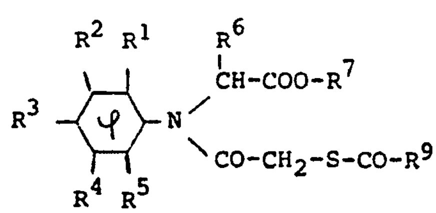

(III)

dadurch gekennzeichnet, daß man ein Mercaptan der Formel

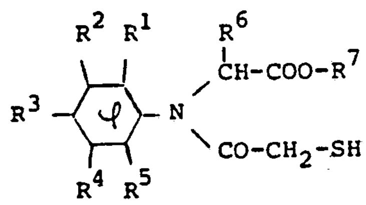

mit einem Chlorid oder Anhydrid der Säure der Formel $R^9$—COOH umsetzt, wobei die Symbole in diesen verschiedenen Formeln derart sind, daß $R^1$ bis $R^7$ und $R^9$ die in einem der Ansprüche 1 bis 5 angebenen Bedeutungen besitzen.

16. Verfahren gemäß Anspruch 15, dadurch gekennzeichnet, daß man ein Säurechlorid der Formel $R^9COCl$ verwendet und daß man die Reaktion in einem inerten Lösungsmittel in Gegenwart eines Säureakzeptors durchführt.

17. Verfahren gemäß Anspruch 15, dadurch gekennzeichnet, daß man ein Säureanhydrid der Formel $(R^9CO)_2O$ verwendet, das in situ mittels der Säure $R^9COOH$ und einem Dehydratisierungsmittel hergestellt wurde.

18. Zusammensetzungen, welche insbesondere zum Schutz von Vegetabilien bzw. Pflanzen gegen Funguskrankheiten verwendbar sind, dadurch gekennzeichnet, daß sie als aktives Material eine Verbindung gemäß einem der Ansprüche 1 bis 5 zusammen mit inerten üblichen Trägern und gegebenenfalls üblichen oberflächenaktiven Mitteln enthalten.

19. Zusammensetzungen gemäß Anspruch 18, dadurch gekennzeichnet, daß sie $5 \cdot 10^{-5}$ bis 95 % aktives Material enthalten.

20. Verfahren zur Bekämpfung von Funguskrankheiten der Pflanzen, dadurch gekennzeichnet, daß man eine Eusammensetzung gemäß einem der Ansprüche 18 oder 19 verwendet.